# DEMANDE DE BREVET EUROPEEN

(11) **EP 4 285 999 A2**
(43) Date de publication de la demande: **06.12.2023**
(21) Numéro de dépôt: 23203108.8
(22) Date de dépôt: 07.04.2017
(51) Int. Cl.: A61P 25/34

(54) **EXTRAIT DE FEUILLES DE TABAC ET UTILISATION POUR LE TRAITEMENT DE L'ADDICTION AU TABAC**

(30) Priorité: 07.04.2016 FR 1653079
(62) Demande divisionnaire de: 17716853.1
(71) Demandeur: NFL Biosciences, 34170 Castelnau-le-Lez (FR)
(72) Inventeur: LAFONT, Bruno, 30170 MONOBLET (FR)
(74) Mandataire: Ipsilon

(57) **Abrégé**

La présente invention concerne un kit comprenant des doses d'extrait de feuilles de tabac ou une composition pharmaceutique comprenant un excipient pharmaceutiquement acceptable et à titre de principe actif un extrait de feuilles de tabac, ledit extrait de feuilles de tabac contenant au moins 5 % en poids, par rapport au poids total de l'extrait sec, de protéines de masse moléculaire supérieure à 10 kDa et étant essentiellement exempt de molécules de masse moléculaire inférieure à 10 kDa.

## Description

### DOMAINE DE L'INVENTION

La présente invention concerne un extrait de feuilles de tabac, ainsi qu'une composition pharmaceutique contenant cet extrait de feuilles de tabac et son utilisation dans le traitement de l'addiction au tabac.

### ART ANTERIEUR

De manière générale, la dépendance ou addiction a été définie par l'Organisation Mondiale de la Santé comme « un syndrome pour lequel la consommation d'un produit devient une exigence supérieure à celles d'autres comportements qui avaient auparavant une plus grande importance. Dans sa forme extrême l'état de dépendance se caractérise par un besoin irrésistible d'un produit qui pousse l'individu souffrant de cette dépendance à la recherche impulsive de ce produit ».

La dépendance au tabac entraîne des problèmes de santé publique important, les maladies étant liées au tabagisme étant très nombreuses tel que le cancer (poumon, gorge, bouche, lèvres...), les maladies cardio-vasculaires, la bronchite chronique... De plus, au-delà des maladies à proprement parler, le tabagisme entraîne de nombreux effets secondaires (diminution de la fertilité, altération de l'épiderme, altération de la muqueuse buccale et nasale, altération des artères cérébrales, atteintes de l'œsophage, de l'estomac, carences en vitamine B et C...).

Il existe trois type de dépendance au tabac qui agissent concomitamment : la dépendance physique qui est due essentiellement à la présence de nicotine dans le tabac et qui se traduit par une sensation de manque (pulsion forte à fumer, irritabilité, nervosité, agitation, anxiété, perturbation du sommeil...) ; la dépendance psychologique qui est liée aux effets psychoactifs de la nicotine qui procure plaisir, détente, stimulation intellectuelle, action anxiolytique, anti dépressive et coupe-faim ; et la dépendance environnementale ou comportementale qui dépend de la pression sociale et conviviale. La dépendance physique disparaît en moyenne en quelques semaines. Les symptômes du sevrage atteignent leur maximum aux alentours de trois à quatre jours après l'arrêt tabagique et peuvent durer quelques semaines. La dépendance psychologique est plus longue à s'estomper et peut durer plusieurs mois.

La dépendance au tabac est maintenue par des renforcements positifs et négatifs. La nicotine est à l'origine de la libération de dopamine, un neurotransmetteur du circuit de la récompense. Le fumeur fume à la fois pour reproduire les sensations de plaisir provoquées par la libération de dopamine (c'est le renforcement positif de la cigarette) et pour éviter les symptômes de manque lorsque que son taux dopamine est trop bas (c'est le renforcement négatif de la cigarette).

Il existe trois méthodes principales de traitement pharmacologique de la dépendance tabagique. La première vise à un arrêt immédiat à partir d'une date d'arrêt cible initialement fixée, les traitements pharmacologiques sont alors administrés après cette date d'arrêt cible dans le cas des substituts nicotiniques et une semaine avant dans le cas du bupropion ou de la varénicline (afin d'atteindre une concentration plasmatique suffisante au moment de la date d'arrêt cible). La seconde consiste à réduire sa consommation avant une tentative d'arrêt, la phase de réduction correspond alors à un prétraitement pendant lequel les traitements pharmacologiques sont administrés. La troisième est celle de la prévention des rechutes après un arrêt réussi. A ces trois méthodes s'ajoute la réduction de la consommation sans pour autant chercher à effectuer une tentative d'arrêt.

L'efficacité de chacune de ces méthodes se trouve renforcée par une phase de préparation du fumeur qui doit se convaincre de la nécessité d'arrêter de fumer et de renforcer sa motivation à y parvenir. Les notices des traitements pharmacologiques de la dépendance tabagique indiquent ainsi que les traitements du sevrage tabagique ont plus de probabilités de succès chez les patients motivés pour arrêter de fumer.

Il existe actuellement quatre grands types de traitement de la dépendance tabagique (sans parler des psychothérapies comportementales, de l'acupuncture ou de l'hypnose), qui sont les substituts nicotiniques, le bupropion, la varénicline et l'homéopathie. Le principe d'action de chacune de ces quatre méthodes est fondé sur le fait que la nicotine est la molécule responsable du mécanisme de la dépendance tabagique.

Les substituts nicotiniques peuvent être administrés de diverses manières : par voie transdermique sous forme de patchs ou timbres, par voie orale sous forme de gommes à mâcher, comprimés à sucer ou comprimés sublinguaux, ou par voie aérienne sous forme d'inhalateur. L'administration de substituts nicotiniques permet principalement la réduction des renforcements négatifs de la cigarette en évitant autant que possible au fumeur de ressentir les symptômes de manque liés au sevrage, la nicotine apportée par les substituts compensant celle des cigarettes. L'efficacité des substituts nicotiniques varient selon la méthode employée, le taux de sevrage étant généralement compris entre 15 à 20 %. Les substituts nicotiniques sont utilisés lors de tentatives d'arrêt immédiat et aussi en prétraitement avant une tentative d'arrêt afin d'aider les fumeurs à réduire leur consommation de cigarettes dans le cadre d'un arrêt graduel (réduire pour arrêter). Ils peuvent aussi être utilisés uniquement dans le but de réduire la consommation de tabac. Leur efficacité dans le cadre de la prévention des rechutes n'a à ce jour pas été démontrée.

Le bupropion commercialisé par les laboratoires GlaxoSmithKline (sous le nom commercial Zyban^{®}), agit sur certains neuromédiateurs cérébraux comme les catécholamines, la noradrénaline et la dopamine. Le bupropion est un inhibiteur sélectif de la recapture neuronale des catécholamines, ce qui lui confère des propriétés d'antidépresseur. Son efficacité est équivalente à celle obtenue après l'utilisation de timbres nicotiniques (taux de sevrage autour de 20 %). Il est moins prescrit que les substituts nicotiniques car il présente un moins bon rapport bénéfices/risques pour une efficacité comparable. Il peut en effet conduire à des tentatives de suicide.

La varénicline commercialisée par les laboratoires Pfizer (sous le nom commercial Chantix^{®} ou Champix^{®}) est un agoniste partiel des récepteurs nicotiniques à l'acéthylcholine. La varenicline cible ces récepteurs avec une double action : agoniste partiel des récepteurs α4β2, elle engendre une réaction similaire à celle induite par la nicotine avec une intensité moindre (et donc réduit les effets de manque lors du sevrage) et en début de traitement quand le fumeur est traité tout en fumant de façon occasionnelle, elle atténue la stimulation neurochimique en présence de nicotine (antagoniste partiel des récepteurs α4β2). La varénicline réduit donc les renforcements négatifs (effet de manque) et positifs (envie de fumer) de la cigarette. En pratique, la varénicline diminue le plaisir que constitue le tabagisme, ce qui serait un critère de futur succès pour arrêter de fumer. Son efficacité est supérieure à celle des substituts nicotiniques (taux de sevrage de l'ordre de 28 %). La varénicline est utilisée lors de tentatives d'arrêt immédiat et aussi en prétraitement pendant quelques semaines avant une tentative d'arrêt afin d'aider les fumeurs à réduire leur consommation de cigarettes dans le cadre d'un arrêt graduel (réduire pour arrêter). Dans une première étude (NCT00789074), 35 % des fumeurs parviennent à réduire d'au moins 50 % leur consommation de cigarettes d'après un point fait après 3 semaines de prétraitement avec la varénicline. Dans une seconde étude (NCT00835900), le pourcentage de réduction du nombre de cigarettes fumées d'après un point fait à 4 semaines de prétraitement avec la varénicline est de 42 %. Dans une troisième étude (NCT01370356), 47 % des fumeurs parviennent à réduire d'au moins 50 % leur consommation de cigarettes d'après un point fait après 4 semaines de prétraitement avec la varénicline. Son efficacité dans le cadre de la prévention des rechutes n'a à ce jour pas été démontrée. Par contre, la varénicline induit des effets secondaires fréquents (nausées) et peut être à l'origine de problèmes cardiaques ou de tentatives de suicide. Ces effets secondaires font qu'en dépit d'une efficacité supérieure à celle des substituts nicotiniques, la varénicline n'est souvent prescrite qu'en seconde intention après un échec avec les substituts nicotiniques.

La quatrième voie de sevrage est l'homéopathie qui repose sur l'utilisation à doses infinitésimales de la substance provoquant les symptômes que l'on désire combattre. Un extrait de « tabacum » est souvent utilisé dans le sevrage tabagique. La demande de brevet irlandaise IE 960 511 décrit notamment l'utilisation de dilutions homéopathiques d'extrait de tabac pour la fabrication d'un médicament destiné à la restauration des fonctions neuronales et au soulagement des symptômes de sevrage de la nicotine. Comme les autres techniques non conventionnelles de sevrage, son efficacité n'est pas suffisante chez les gros fumeurs.

Malgré les nombreuses recherches dans ce domaine, il existe toujours la nécessité de trouver de nouveaux traitements ou d'améliorer les traitements existants permettant de traiter la dépendance tabagique.

Dans la demande PCT/FR2007/000786, la Demanderesse avait découvert que l'injection chez un fumeur d'une solution aqueuse d'extrait de feuilles tabac permettait de traiter la dépendance au tabac dudit fumeur. Cette découverte était réellement inattendue car la Demanderesse avait pu déterminer que ledit extrait aqueux de feuilles de tabac contenait de faibles quantités de nicotine. Ainsi, le mécanisme d'action impliqué dans le traitement de la dépendance tabagique à l'aide de cet extrait aqueux de feuilles de tabac n'est pas l'apport de nicotine au fumeur.

Néanmoins le fait que ledit extrait aqueux de feuilles de tabac contenait de faibles quantités de nicotine ne renseignait pas sur sa composition et son mode de fabrication. La teneur en nicotine des extraits aqueux de feuilles de tabac peut en effet varier considérablement en fonction de nombreux facteurs.

La teneur en nicotine dans les feuilles de tabac peut ainsi varier de 0,5 à 8 % pour les principaux types de tabac cultivés (Davis et Al., "Production, Chemistry, and Technology", ISBN-13: 978-0632047918, Chapter 8, page 275).

Le tabac Burley contient ainsi environ 2 fois plus de nicotine que le tabac Marynland et 3 fois plus de nicotine que le tabac Oriental (Leffingwell, « Chemical constituents of tobacco leaf and différences among tobacco types », Leffingwell Reports, Vol. 1 (No. 2), February, 2001).

L'utilisation d'engrais azotés influence la teneur en nicotine des feuilles de tabac et la teneur en nicotine est plus importante dans les feuilles de l'étage haut que dans celles de l'étage intermédiaire qui est elle-même plus importante que dans celles de l'étage bas (Xiao-Tang et al., « Yield and nicotine content of flue-cured tobacco as affected by soit nitrogen mineralization », Pedosphere 18(2): 227-235, 2008, ISSN 1002-0160/CN 32-1315/P).

La rotation des cultures influence la teneur en nicotine des feuilles de tabac (Butorac et al., « The Effect of Tobacco Monoculture and Crop Rotations on Tobacco Leaf Composition », Agriculturae Conspectus Scientificus, Vol. 69 (2004) No. 4 (95-101)). La composition chimique et la teneur en nicotine des feuilles de tabac sont influencées par les rayonnement ultra-violet et l'intensité lumineuse (Andersen et al., « Chemical composition of tobacco leaves altered by near-ultraviolet and intensity of visible light », Plant Physiol. (1973) 51, 723-726).

Les méthodes de préparation des extraits influencent aussi la teneur en nicotine, notamment les temps et conditions de macération, les méthodes de stérilisation et les séparations physiques et chimiques.

Un extrait de tabac tel que celui décrit dans la demande PCT/FR2007/000786 comprend des molécules de faible masse moléculaire, i.e. inférieure à 10 kDa. Parmi ces molécules de faible poids moléculaire, on peut notamment citer les N-nitrosamines (TSNA) et les N-nitrosoamino acides spécifiques au tabac dérivés d'alcaloïdes non-volatiles, des aldéhydes volatiles, des hydrocarbures aromatiques polynucléaires (tel que le benzo[a]pyrène), des lactones, des uréthanes, ou des métaux tel que le cadmium par exemple (cf. Wagner et al., Plant Physiol. 1986, 82, 274-279 et IARC Monographs on the Evaluation of Carcinogenic Risks to Human, vol.49). Ces composés peuvent être carcinogènes, mutagènes ou toxiques et peuvent provoquer des cancers, des problèmes rénaux ou osseux. Ces composés sont donc mauvais pour la santé du patient auquel on a administré une composition les comprenant.

Un tel extrait de tabac comprend de nombreuses protéines de tout poids moléculaire. La base de données SwissProt identifie ainsi 759 protéines de tabac. La protéine RuBisCO peut représenter à elle seule de 30 à 50 % des protéines solubles. Une protéine RuBisCO complète compte typiquement huit sous-unités formant un complexe protéique d'environ 540 kDa. La toxicité de ces protéines administrées en sous-cutané dans une solution les comprenant est inconnue alors que rien n'indique qu'elles puissent toutes ou certaines être impliquées dans un quelconque effet thérapeutique. L'objectif de la présente invention est de fournir un traitement de l'addiction au tabac qui soit efficace. De manière avantageuse, ce traitement n'est pas toxique, est bien toléré par les patients et présente un risque limité en réduisant autant que possible l'administration de molécules à la toxicité inconnue et qui ne présentent *a priori* pas d'effet thérapeutique.

Alors que les extraits de tabac contiennent des milliers de composés, la Demanderesse a découvert de manière surprenante que des protéines des extraits de feuilles de tabac permettent d'induire une réponse immunitaire de type IgG spécifique qui favorise le sevrage tabagique. Ainsi, l'extrait de feuilles de tabac selon l'invention comprenant une teneur élevée en protéines et une composition protéique particulière permet d'induire une réponse immunitaire de type IgG spécifique qui favorise le sevrage tabagique.

Cette découverte est d'autant plus surprenante que les extraits aqueux de feuilles de tabac contiennent des milliers de composés, les protéines ne constituant qu'une famille de composés parmi d'autres. Les protéines de tabac ne sont pas connues pour jouer un rôle dans l'addiction au tabac et rien n'incite donc à les utiliser dans le sevrage tabagique.

La Demanderesse montre notamment que les extraits de feuilles de tabac selon l'invention présentent une teneur élevée en protéines et une composition protéique particulière. Cette composition protéique particulière peut être notamment obtenue lorsque des feuilles de tabac séchées sont utilisées. Le séchage favorise la dégradation des protéines, par exemple par des mécanismes d'hydrolyse, et l'augmentation des acides aminés libres dans les feuilles de tabac (Hamilton & Lowe, 1978 ; Long & Weybrew, 1981 ; Burton, et al., 1983).

La Demanderesse a découvert de manière surprenante que des protéines des extraits de feuilles de tabac selon l'invention permettent d'induire la génération d'anticorps IgG spécifiques qui favorisent le sevrage tabagique. La Demanderesse montre que l'administration de l'extrait de feuilles de tabac selon l'invention induit la réponse immunitaire de type IgG spécifique et permet d'obtenir le traitement efficace de l'addiction au tabac, même sans ajouter à l'extrait d'adjuvant pouvant favoriser une réponse immunitaire. La Demanderesse montre que la réponse immunitaire de type IgG spécifique, le traitement de l'addiction au tabac et le sevrage tabagique sont particulièrement efficaces lorsque des extraits de feuilles de tabac séchées sont utilisés.

Cette découverte est d'autant plus surprenante que rien dans l'art antérieur n'indique que des anticorps IgG spécifiques des protéines d'extraits de feuilles de tabac sont susceptibles d'aider les fumeurs à réduire ou à arrêter leur consommation tabagique. L'activation d'une réponse immunitaire de type IgG spécifique suite à l'administration d'extraits de tabac, et le rôle d'extraits de tabac dans le sevrage tabagique n'avait jamais été décrit à ce jour. Un tel lien entre le système immunitaire (impliqué notamment dans la production des anticorps IgG spécifiques), et le système nerveux, (impliqué notamment dans la perception de l'acte de fumer et le comportement) pouvant favoriser le sevrage tabagique n'a jamais été décrit à ce jour.

De manière avantageuse, le traitement de l'addiction au tabac selon la présente invention nécessite l'administration de la composition pharmaceutique selon l'invention que peu de fois, avantageusement une seule administration, pour que des effets des renforcements de la cigarette ne soient plus ressentis ou ressentis de façon significativement atténuée par le patient, et notamment l'envie de fumer (renforcement positif), l'appétence de la cigarette est réduite.

### RESUME DE L'INVENTION

Dans un premier aspect, la présente invention concerne un extrait de feuilles de tabac contenant au moins 5 % en poids, par rapport au poids total de l'extrait sec, de protéines de masse moléculaire supérieure à 10 kDa et essentiellement exempt de molécules de masse moléculaire inférieure à 10 kDa. De préférence, lesdites protéines sont choisies parmi le groupe constitué des familles de protéines suivantes : peroxidase anionique formant de la lignine, glucan endo-1,3-béta-glucosidase, endochitinase, protéine liée à la pathogénèse, osmotine et inhibiteur de protéinase ainsi que leurs mélanges.

L'invention concerne en outre une composition pharmaceutique comprenant ledit extrait de feuilles de tabac et son utilisation dans le traitement de l'addiction au tabac.

L'invention concerne en outre une composition pharmaceutique comprenant ledit extrait de feuilles de tabac et son utilisation dans le sevrage tabagique.

L'invention concerne en outre une méthode de traitement de l'addiction au tabac comprenant l'administration dudit extrait de feuilles de tabac à un sujet souffrant d'addiction ou de dépendance au tabac.

L'invention concerne en outre une méthode de traitement de l'addiction au tabac comprenant l'administration de ladite composition pharmaceutique comprenant ledit extrait de feuilles de tabac à un sujet souffrant d'addiction au tabac.

Dans le cadre de la présente invention, le traitement de la dépendance ou de l'addiction au tabac englobe toutes les formes de consommation de tabacs, à savoir les tabacs fumés, sous forme de cigarettes manufacturées ou roulées, de cigarillos ou cigares, de tabacs fumés dans la pipe, mais aussi des tabacs non fumés tels que des tabacs à priser, chiquer ou sucer.

L'invention concerne également le procédé de préparation dudit extrait de feuilles de tabac contenant au moins 5 % en poids, par rapport au poids total de l'extrait sec, de protéines de masse moléculaire supérieure à 10 kDa et essentiellement exempt de molécules de masse moléculaire inférieure à 10 kDa comprenant les étapes suivantes :
a. Séchage de feuilles de tabac,
b. Broyage de feuilles de tabac séchées pour obtenir un broyat de feuilles de tabac séchées,
c. Extraction du broyat de feuilles de tabac séchées sous agitation mécanique avec un solvant, par exemple un solvant aqueux, de préférence une solution tampon aqueuse à pH compris entre 6,0 et 8,5,
d. Séparation des résidus solides de la solution de l'extrait du broyat de feuilles de tabac séchées par filtration ou centrifugation afin d'obtenir une solution d'extrait de broyat de feuilles de tabac séchées sans résidu solide,
e. Diafiltration à volume constant de la solution obtenue à l'étape d avec un solvant, de préférence un solvant aqueux, en une quantité allant de 2 à 12 fois en volume, de préférence de 3 à 10 fois en volume, de préférence de 4 à 8 fois en volume, de préférence 6 fois en volume, par rapport au volume de l'extrait, et avec une membrane ayant un seuil de coupure à 10 kDa,
f. Eventuellement lyophilisation de la solution protéique obtenue à l'étape e.

L'invention concerne également un kit comprenant une composition pharmaceutique comprenant ledit extrait de feuilles de tabac contenant au moins 5 % en poids, par rapport au poids total de l'extrait sec, de protéines de masse moléculaire supérieure à 10 kDa et essentiellement exempt de molécules de masse moléculaire inférieure à 10 kDa.

### Résumé descriptif de l'invention sous forme de clauses :

Clause 1 : Extrait de feuilles de tabac contenant au moins 5 % en poids, par rapport au poids total de l'extrait sec, de protéines de masse moléculaire supérieure à 10 kDa et essentiellement exempt de molécules de masse moléculaire inférieure à 10 kDa, lesdites protéines étant de préférence choisies parmi le groupe constitué des familles de protéines suivantes : peroxidase anionique formant de la lignine, glucan endo-1,3-béta-glucosidase, endochitinase, protéine liée à la pathogénèse, osmotine et inhibiteur de protéinase ainsi que leurs mélanges.

Clause 2. Extrait de feuilles de tabac selon la clause 1, caractérisé en ce qu'il est essentiellement exempt de protéines de haute masse moléculaire.

Clause 3 : Extrait de feuilles de tabac selon la clause 1 ou la clause 2, caractérisé en ce qu'il est essentiellement exempt de protéines dont la masse moléculaire est supérieure à 500 kDa, de préférence dont la masse moléculaire est supérieure à 400 kDa, de préférence encore dont la masse moléculaire est supérieure à 300 kDa, de manière préférentielle dont la masse moléculaire est supérieure à 200 kDa, de manière plus préférentielle dont la masse moléculaire est supérieure à 150 kDa, de manière plus préférentielle encore dont la masse moléculaire est supérieure à 100 kDa et mieux encore dont la masse moléculaire est supérieure à 50 kDa.

Clause 4 : Extrait de feuilles de tabac selon l'une quelconque des clauses précédentes, caractérisé en ce que la teneur en molécules de masse moléculaire inférieure à 10 kDa est inférieure à 5 % en poids par rapport au poids total de l'extrait, de préférence inférieure 2,5 % en poids, et mieux encore inférieure à 1 % en poids.

Clause 5 : Extrait de feuilles de tabac selon l'une quelconque des clauses précédentes, caractérisé en ce qu'il est susceptible d'être obtenu par un procédé d'extraction par un solvant, par exemple un solvant aqueux, d'un broyât de feuilles de tabac séchées suivie d'une séparation des résidus solides de la solution d'extrait de broyât de feuilles de tabac séchées puis d'une diafiltration à volume constant de la solution d'extrait de broyât de feuilles de tabac séchées sans résidu solide avec un solvant aqueux en une quantité allant de 2 à 12 fois en volume, de préférence 3 à 10 fois en volume, de préférence 4 à 8 fois en volume, de préférence 6 fois en volume, par rapport au volume de l'extrait et avec une membrane ayant un seuil de coupure à 10 kDa.

Clause 6 : Extrait de feuilles de tabac selon l'une quelconque des clauses précédentes, caractérisé en ce qu'il comprend au moins une protéine appartenant à la famille des glucan endo-1,3-béta-glucosidases, et de préférence choisie(s) parmi l'isoforme acide PR-Q' de la béta-1,3- endoglucanase (PR36401 selon la base UniProt), l'isoforme vacuolaire basique GLB de la béta-1,3-endoglucanase (P27666 selon la base UniProt), et leurs mélanges.

Clause 7 : Extrait de feuilles de tabac selon l'une quelconque des clauses précédentes, caractérisé en ce qu'il comprend au moins une protéine appartenant à la famille des endochitinases, et de préférence choisie(s) parmi l'endochinitase P acide (P17513 selon la base UniProt), l'endochinitase Q acide (P17514 selon la base UniProt), l'endochinitase B (P24091 selon la base UniProt), et leurs mélanges.

Clause 8 : Extrait de feuilles de tabac selon l'une quelconque des clauses précédentes, caractérisé en ce qu'il comprend au moins de l'osmotine (P14170 selon la base UniProt).

Clause 9 : Extrait de feuilles de tabac selon l'une quelconque des clauses précédentes, caractérisé en ce qu'il comprend au moins une peroxidase anionique formant de la lignine (P11965 selon la base UniProt).

Clause 10 : Extrait de feuilles de tabac selon l'une quelconque des clauses précédentes, caractérisé en ce qu'il comprend au moins une protéine liée à la pathogénèse, et de préférence choisie(s) parmi la protéine R liée à la pathogénèse (P13046 selon la base UniProt), la protéine PR-4A liée à la pathogénèse (PR29062 selon la base UniProt), la protéine PR-4B liée à la pathogénèse (PR29063 selon la base UniProt), et leurs mélanges.

Clause 11 : Extrait de feuilles de tabac selon l'une quelconque des clauses précédentes, caractérisé en ce qu'il comprend au moins une protéine appartenant à la famille des inhibiteurs de protéinase, et de préférence choisie(s) parmi l'inhibiteur de protéinase I-B (Q03199 selon la base UniProt), l'inhibiteur de protéinase I-A (Q03198 selon la base UniProt), et leurs mélanges.

Clause 12 : Extrait de feuilles de tabac selon au moins l'une quelconque des clauses précédentes, caractérisé en ce que la teneur en protéines dans l'extrait sec est d'au moins 10 % en poids, de préférence au moins 15 % en poids, de préférence au moins 20 % en poids par rapport au poids total de l'extrait sec.

Clause 13 : Composition pharmaceutique comprenant à titre de principe actif un extrait de feuilles de tabac selon l'une quelconque des clauses précédentes ainsi qu'un excipient pharmaceutiquement acceptable.

Clause 14 : Composition pharmaceutique selon la clause 13, caractérisée en ce que les protéines présentes dans ledit extrait de feuilles de tabac sont présentes en une quantité allant de 1 à 1000 µg/mL, de préférence de 10 à 500 µg/mL, de préférence de 50 à 300 µg/mL, de préférence de 60 à 200 µg/mL, de préférence entre 80 et 150 µg/mL.

Clause 15 : Composition pharmaceutique selon la clause 13 ou la clause 14, caractérisée en ce qu'elle contient en outre des protéines extraites du cannabis.

Clause 16 : Composition pharmaceutique selon l'une quelconque des clauses 13 à 15, caractérisée en ce qu'elle est présentée sous une forme propre à une administration par injection sous cutanée.

Clause 17 : Composition pharmaceutique selon l'une quelconque des clauses 13 à 16 pour son utilisation dans le traitement de l'addiction au tabac.

Clause 18 : Composition pharmaceutique selon la clause 15, pour son utilisation dans le traitement conjoint de l'addiction au tabac et au cannabis.

Clause 19 : Composition pharmaceutique pour son utilisation dans le traitement de l'addiction au tabac selon la clause 17, ou dans le traitement conjoint de l'addiction au tabac et au cannabis selon la clause 18, caractérisée en ce que la composition pharmaceutique est présentée sous une forme de dosage de 0,03 mL à 10 mL, de préférence de 0,1 mL à 5 mL, de préférence de 0,5 à 2 mL.

Clause 20 : Procédé de préparation d'un extrait de feuilles de tabac selon l'une quelconque des clauses 1 à 12, comprenant les étapes suivantes :
a. Séchage de feuilles de tabac,
b. Broyage de feuilles de tabac séchées pour obtenir un broyât de feuilles de tabac séchées,
c. Extraction du broyât de feuilles de tabac séchées sous agitation mécanique avec un solvant, par exemple un solvant aqueux, de préférence une solution tampon aqueuse à pH compris entre 6,0 et 8,5,
d. Séparation des résidus solides de la solution de l'extrait du broyât de feuilles de tabac séchées par filtration ou centrifugation afin d'obtenir une solution d'extrait de broyât de feuilles de tabac séchées sans résidu solide,
e. Diafiltration à volume constant de la solution obtenue à l'étape c avec un solvant, de préférence un solvant aqueux, en une quantité allant de 2 à 12 fois en volume, de préférence 3 à 10 fois en volume, de préférence de 4 à 8 fois en volume, de préférence 6 fois en volume, par rapport au volume de l'extrait, et avec une membrane ayant un seuil de coupure à 10 kDa,
f. Eventuellement lyophilisation de la solution protéique obtenue à l'étape d.

### DESCRIPTION DES FIGURES

**Figure 1****.** Illustration d'un profil électrophorétique de l'extrait de feuilles de tabac selon l'exemple 1 : Fractionnement en gel NuPage 4 % - 12 % en tampon MES et en présence d'agent réducteur.
**Figure 2****.** Induction d'IgG après injection de l'extrait de feuilles de tabac selon l'exemple 1 à des souris.
**Figure 3****.** Illustration d'un immunophénotypage pour la caractérisation de cellules immunes activées.
**Figure 4****.** Activation des cellules Natural Killer par l'extrait de feuilles de tabac selon l'exemple 1.
**Figure 5****.** Activité de l'extrait de feuilles de tabac selon l'exemple 1 sur les lymphocytes T et B.
**Figure 6****.** Induction de cytokines pro-inflammatoires et d'FNy par l'extrait de feuilles de tabac selon l'exemple 1.
**Figure 7****.** Induction de cytokines TH2 et de chimiokines et facteur de croissance hématopoïétique par l'extrait de feuilles de tabac selon l'exemple 1.
**Figures 8 et 9****.** Evolution de la consommation de tabac chez des patients traités par l'administration de l'extrait de feuilles de tabac selon l'exemple .1

### DESCRIPTION DETAILLEE DE L'INVENTION

### Extrait de feuilles de tabac

La présente invention concerne un extrait de feuilles de tabac contenant au moins 5 % en poids, par rapport au poids total de l'extrait sec, de préférence au moins 10 % en poids, de préférence au moins 15 % en poids, de préférence environ 20 % en poids de protéines de masse moléculaire supérieure à 10 kDa et essentiellement exempt de molécules de masse moléculaire inférieure à 10 kDa.

L'extrait sec est avantageusement obtenu avant lyophilisation de l'extrait de feuilles de tabac selon l'invention, puis mise sous cloche à vide, de préférence en présence de P₂O₅, jusqu'à obtention d'une masse d'extrait constante.

De manière avantageuse, la teneur en molécules de masse moléculaire inférieure à 10 kDa est inférieure à 5 % en poids par rapport au poids total de l'extrait, de préférence inférieure 2,5 % en poids, et mieux encore inférieure à 1 % en poids.

Au sens de la présente invention, on entend par « environ » plus ou moins 1 % en raison des incertitudes de mesure.

La teneur en protéines de masse moléculaire supérieure à 10 kDa habituellement mesurée dans des extraits de feuilles de tabac est d'environ 1 %. L'extrait de feuilles de tabac selon la présente invention a donc une teneur en protéines de masse moléculaire supérieure à 10 kDa bien plus élevée que ce qui était connu jusque-là.

De préférence, les protéines présentes dans l'extrait selon la présente invention sont choisies parmi le groupe constitué des familles de protéines suivantes : peroxidase anionique formant de la lignine, glucan endo-1,3-béta-glucosidase, endochitinase, protéine liée à la pathogénèse, osmotine et inhibiteur de protéinase ainsi que leurs mélanges.

Les noms indiqués pour les protéines présentes dans l'extrait selon la présente invention correspondent aux noms renseignés dans la banque de données Swissprot qui est une banque de données biologiques répertoriant les séquences protéiques.

Selon un mode de réalisation, l'extrait de feuilles de tabac selon l'invention comprend au moins une protéine appartenant à la famille des glucan endo-1,3-béta-glucosidases, et de préférence choisie(s) parmi l'isoforme acide PR-Q' de la béta-1,3-endoglucanase (PR36401 selon la base UniProt), l'isoforme vacuolaire basique GLB de la béta-1,3-endoglucanase (P27666 selon la base UniProt), et leurs mélanges.

Selon un mode de réalisation, l'extrait de feuilles de tabac selon l'invention comprend au moins une protéine appartenant à la famille des endochitinases, et de préférence choisie(s) parmi l'endochinitase P acide (P17513 selon la base UniProt), l'endochinitase Q acide (P17514 selon la base UniProt), l'endochinitase B (P24091 selon la base UniProt), et leurs mélanges.

Selon un mode de réalisation, l'extrait de feuilles de tabac selon l'invention comprend au moins de l'osmotine (P14170 selon la base UniProt).

Selon un mode de réalisation, l'extrait de feuilles de tabac selon l'invention comprend au moins une peroxidase anionique formant de la lignine (P11965 selon la base UniProt).

Selon un mode de réalisation, l'extrait de feuilles de tabac selon l'invention comprend au moins une protéine liée à la pathogénèse, et de préférence choisie(s) parmi la protéine R liée à la pathogénèse (P13046 selon la base UniProt), la protéine PR-4A liée à la pathogénèse (PR29062 selon la base UniProt), la protéine PR-4B liée à la pathogénèse (PR29063 selon la base UniProt), et leurs mélanges.

Selon un mode de réalisation, l'extrait de feuilles de tabac selon la présente invention comprend au moins une protéine appartenant à la famille des inhibiteurs de protéinase, et de préférence choisie(s) parmi l'inhibiteur de protéinase I-B (Q03199 selon la base UniProt), l'inhibiteur de protéinase I-A (Q03198 selon la base UniProt), et leurs mélanges.

Selon un mode de réalisation, l'extrait de feuilles de tabac comprend également des polysaccharides de masse moléculaire supérieure à 10 kDa et de préférence hydrosolubles.

De préférence, l'extrait de feuilles de tabac selon la présente invention comprend au moins 5 % en poids par rapport au poids total de l'extrait sec, de préférence au moins 10 % en poids, de préférence au moins 15 % en poids, de préférence environ 20 % en poids de protéines choisies parmi le groupe constitué des familles de protéines suivantes : peroxidase anionique formant de la lignine, glucan endo-1,3-béta-glucosidase, endochitinase, protéine liée à la pathogénèse, osmotine et inhibiteur de protéinase ainsi que leurs mélanges.

Selon un mode de réalisation, l'extrait de feuilles de tabac selon la présente invention est essentiellement exempt de protéines de haute masse moléculaire.

Au sens de la présente invention, on entend par « protéine de haute masse moléculaire » une protéine dont la masse moléculaire est supérieure à 500 kDa de préférence dont la masse moléculaire est supérieure à 400 kDa, de préférence encore dont la masse moléculaire est supérieure à 300 kDa, de manière préférentielle dont la masse moléculaire est supérieure à 200 kDa, de manière plus préférentielle encore dont la masse moléculaire est supérieure à 150 kDa, et mieux encore dont la masse moléculaire est supérieure à 100 kDa.

Selon un mode de réalisation, l'extrait de feuilles de tabac selon la présente invention est essentiellement exempt de protéines dont la masse moléculaire est supérieure à 50 kDa.

Au sens de la présente invention, on entend par « essentiellement exempt » une teneur en molécules inférieure à 15 % en poids par rapport au poids protéique total de l'extrait, de préférence une teneur en molécules inférieure à 10 % en poids par rapport au poids protéique total de l'extrait, de préférence encore inférieure à 7,5 % en poids par rapport au poids protéique total de l'extrait, de préférence encore inférieure à 5 % en poids par rapport au poids protéique total de l'extrait, de manière préférentielle inférieure 2,5 % en poids par rapport au poids protéique total de l'extrait, de manière plus préférentielle encore inférieure 1 % en poids par rapport au poids protéique total de l'extrait, et mieux encore inférieure à 0,5 % en poids par rapport au poids protéique total de l'extrait.

De manière avantageuse, la teneur en protéines de haute masse moléculaire est inférieure à 15 % en poids par rapport au poids protéique total de l'extrait, de préférence inférieure à 10 % en poids par rapport au poids protéique total de l'extrait, de préférence encore inférieure à 7,5 % en poids par rapport au poids protéique total de l'extrait, de préférence encore inférieure à 5 % en poids par rapport au poids protéique total de l'extrait, de manière préférentielle inférieure 2,5 % en poids par rapport au poids protéique total de l'extrait, de manière plus préférentielle encore inférieure 1 % en poids par rapport au poids protéique total de l'extrait, et mieux encore inférieure à 0,5 % en poids par rapport au poids protéique total de l'extrait.

Selon un mode de réalisation, l'extrait de feuilles de tabac selon la présente invention est essentiellement exempt de protéines RuBisCO.

Selon un mode de réalisation, les feuilles de tabac sont de la variété *Nicotiana Tabacum* ou de la variété *Nicotiana Rustica.* Les feuilles de tabac peuvent provenir d'un tabac brun ou blond et peut être notamment choisi parmi le tabac de Virginie, le tabac Burley, le tabac oriental, le tabac Latakia, le tabac Périque, le tabac Maryland, le tabac Kentucky, le tabac de Californie, le tabac Tex Mex, et leurs mélanges.

Bien entendu, l'extrait n'est pas nécessairement constitué par un extrait pur de feuilles de tabac et des protéines extraites du cannabis, peuvent être ajoutées pour traiter conjointement la dépendance au tabac et au cannabis.

Selon un mode particulier de réalisation, l'extrait de feuilles de tabac est obtenu à partir d'un mélange 1 /1 /1 de tabac brun, de tabac de Virginie et de Burley.

Selon un mode de réalisation particulier, l'extrait de feuilles de tabac est obtenu à partir de tabac Burley.

Selon un mode de réalisation, l'extrait de feuilles de tabac est obtenu à partir de feuilles de tabac séchées.

Selon un mode de réalisation particulier, l'extrait de feuilles de tabac est obtenu à partir de feuilles de tabac séchées à l'air libre.

Selon un mode de réalisation particulier, l'extrait de feuilles de tabac est obtenu à partir de feuilles de tabac séchées à l'air libre pour une durée adaptée aux conditions climatiques locales, de préférence pour une durée minimum d'un mois.

Au sens de la présente invention, on entend par « séchage à l'air libre » ou « séchage naturel » un séchage réalisé en présence de l'air libre naturel, à l'extérieur ou à l'intérieur. Le séchage à l'air libre peut être réalisé dans un local ouvert ou fermé, couvert ou non. Un séchage à l'air libre peut par exemple être réalisé en séchoir naturel. Dans ce cas, les feuilles de tabac fraichement récoltées ou préséchées sont laissées à sécher de manière naturelle sous l'effet de l'air libre. Les feuilles de tabac peuvent par exemple être suspendues dans des hangars ventilés non chauffés. Les feuilles de tabac peuvent être par exemple laissées à sécher naturellement jusqu'à ce qu'elles prennent une couleur brune. A ce stade, il n'y a pratiquement plus de sucre dans la feuille. Avantageusement, le séchage est réalisé pour une durée adaptée aux conditions climatiques locales, de préférence pour une durée minimum d'un mois. A titre d'exemple, le séchage peut être réalisé entre septembre et décembre pour les récoltes du centre de la France. Les feuilles de tabac peuvent être retournées ou aérées une ou plusieurs fois durant le séchage, de manière à permettre un séchage homogène et éviter la formation de condensation, le pourrissement ou la dégradation des feuilles. La méthode de séchage à l'air libre peut être utilisée par exemple pour le tabac de variété Burley.

Selon un mode de réalisation particulier, l'extrait de feuilles de tabac est obtenu à partir de feuilles de tabac séchées en séchoir naturel.

Selon un autre mode de réalisation particulier, l'extrait de feuilles de tabac est obtenu à partir de feuilles de tabac séchées en séchoir chauffant. Dans ce cas, le séchage peut être réalisé dans un hangar ou un local chauffé à une température adaptée.

Selon un autre mode de réalisation particulier, l'extrait de feuilles de tabac est obtenu à partir de feuilles de tabac séchées par séchage au four. Dans ce cas, le séchage peut être réalisé dans un local ou un hangar chauffé à une température adaptée. La chaleur peut être introduite dans le local ou le hangar par des conduits reliés à une chaudière extérieure. Ce chauffage contrôlé permet d'obtenir des feuilles d'une couleur jaune-orange. Ces feuilles contiennent alors un fort taux de sucre. Le tabac de Virginie peut par exemple être séché selon cette méthode.

Selon un autre mode de réalisation particulier, l'extrait de feuilles de tabac est obtenu à partir de feuilles de tabac séchées par séchage au soleil. Dans ce cas, les feuilles de tabac peuvent être étendues sur des claies et exposées au soleil pendant 12 à 30 jours. Sous la chaleur et le rayonnement directs du soleil, les feuilles prennent une couleur jaune ou orange et gardent un fort taux de sucre. Le tabac oriental est généralement séché selon cette méthode.

Selon un autre mode de réalisation particulier, l'extrait de feuilles de tabac est obtenu à partir de feuilles de tabac séchées par séchage au feu. Dans ce cas, on peut brûler du petit bois sous les feuilles de tabac, qui sèchent en s'imprégnant d'un arôme « fumé ».

L'extrait de feuilles de tabac selon la présente invention est avantageusement un extrait aqueux.

L'extrait de feuilles de tabac selon la présente invention est avantageusement un extrait aqueux de couleur brune.

De préférence, l'extrait de feuilles de tabac est susceptible d'être obtenu par un procédé d'extraction par un solvant, par exemple un solvant aqueux, d'un broyat de feuilles de tabac séchées suivie d'une séparation des résidus solides de la solution d'extrait de broyat de feuilles de tabac séchées puis d'une diafiltration à volume constant de la solution d'extrait de broyat de feuilles de tabac séchées sans résidu solide avec un solvant aqueux en une quantité allant de 2 à 12 fois en volume, de préférence 3 à 10 fois en volume, de préférence 4 à 8 fois en volume, de préférence 6 fois en volume, par rapport au volume de l'extrait et avec une membrane ayant un seuil de coupure à 10 kDa.

Bien entendu, d'autres types de solvant peuvent être utilisés pour réaliser le procédé d'extraction de l'extrait de feuilles de tabac. L'extraction de l'étape c peut être réalisée avec un solvant organique ou inorganique, ou avec un mélange de solvants organiques et/ou inorganiques.

Pour assurer une bonne efficacité et un bon rendement d'extraction, et pour obtenir un extrait de bonne qualité présentant les propriétés de pureté et la composition désirées, l'homme du métier saura aisément sélectionner le ou les solvants appropriés selon les critères suivants :
- la polarité du solvant ou du mélange de solvant (polaire ou apolaire) ;
- l'état physique du solvant ou du mélange de solvant (par exemple liquide, solide, supercritique, ou gazeux) ;
- la nature chimique du solvant ou du mélange de solvant (par exemple organique ou inorganique) ;
- la charge du solvant ou du mélange de solvant (par exemple ionique ou non ionique) ;
- l'origine du solvant ou du mélange de solvant ;
- la miscibilité du solvant ou du mélange de solvant ;
- la solubilité du solvant ou du mélange de solvant.

L'homme du métier saura quelle technique utiliser pour sélectionner le ou les solvants correspondant à ses critères, en fonction du rendement d'extraction, de la pureté et de la composition de l'extrait désirés.

Avantageusement, l'extrait de feuilles de tabac selon la présente invention est obtenu par le procédé de préparation de feuilles de tabac décrit ci-après.

### Composition pharmaceutique

Dans un deuxième aspect, la présente invention concerne une composition pharmaceutique comprenant à titre de principe actif l'extrait de feuilles de tabac tel que décrit ci-dessus.

La composition pharmaceutique selon la présente invention comprend en outre au moins un excipient pharmaceutiquement acceptable, tel que les solvants pharmaceutiquement acceptables, et par exemple de l'eau.

Avantageusement, la composition pharmaceutique comprend des protéines présentes dans l'extrait de feuilles de tabac selon l'invention en une teneur allant de 1 à 1 000 µg/mL, de préférence de 10 à 500 µg/mL, de préférence de 50 à 300 µg/mL, de préférence de 60 à 200 µg/mL, de préférence entre 80 et 150 µg/mL.

La composition pharmaceutique selon l'invention est de préférence une composition aqueuse.

Selon un mode de réalisation particulier, la composition pharmaceutique peut comprendre en outre un actif agissant sur le renforcement négatif, tel que par exemple un substitut nicotinique, la varenicline ou le bupropion.

Selon un mode de réalisation, la composition pharmaceutique comprend en outre un adjuvant. On peut notamment citer comme adjuvant les agents de gonflement comme par exemple un sucre tel que le lactose, le saccharose, le tréhalose, le sorbitol, le glucose, le raffinose, le mannitol, de préférence le lactose, le saccharose, le tréhalose, le glucose, ou le mannitol, un acide aminé tel que l'arginine, la glycine, ou l'histidine, de préférence la glycine, ou des polymères de type dextrane ou polyéthylène glycol, ou leurs mélanges. Selon ce mode de réalisation, la composition pharmaceutique comprend 50 à 99 % en poids d'adjuvants, de préférence de 80 à 97 % en poids, par rapport au poids total de la composition pharmaceutique.

Selon un mode de réalisation particulier, la composition pharmaceutique peut comprendre en outre des protéines extraites du cannabis.

Selon un mode de réalisation, la composition pharmaceutique selon la présente invention est présentée sous une forme propre à une administration sous cutanée.

Selon un autre mode de réalisation, la composition pharmaceutique est présentée sous une forme propre à une administration à l'aide d'un système thérapeutique transdermique adhésif. Avantageusement, la composition pharmaceutique est présentée sous une forme de patch.

Selon un autre mode de réalisation, la composition pharmaceutique est présentée sous une forme propre à une administration par pulvérisation ou par vaporisation. Avantageusement, la composition pharmaceutique est présentée sous une forme de pulvérisateur, de vaporisateur, ou de spray.

La composition pharmaceutique selon l'invention est de préférence préparée selon le procédé de préparation de composition pharmaceutique comprenant l'extrait de feuilles de tabac décrit ci-après.

### Utilisation de la composition pharmaceutique

Dans un troisième aspect, la présente invention concerne l'utilisation de la composition pharmaceutique pour le traitement de l'addiction au tabac.

Par « traitement de l'addiction au tabac » ou par « traitement de la dépendance au tabac », on entend plus précisément au sens de la présente invention la réduction des renforcements addictifs de la cigarette. L'administration de la composition pharmaceutique selon l'invention permet notamment de réduire les renforcements positifs de la cigarette et notamment l'envie de fumer.

La présente invention concerne également une composition comprenant un extrait de feuilles de tabac tel que défini ci-dessus pour son utilisation dans le traitement de l'addiction au tabac.

En d'autres termes, la présente invention concerne également une méthode pour traiter l'addiction ou la dépendance au tabac, comprenant l'administration d'une composition comprenant un extrait de feuilles de tabac tel que défini ci-dessus.

Avantageusement, l'administration de la composition selon l'invention permet au patient d'arrêter de fumer, de diminuer sa consommation ou de prévenir sa rechute après un arrêt en atténuant les renforcements positifs du tabac et notamment l'envie de fumer, facilitant ainsi le sevrage tabagique. Avantageusement, l'administration de la composition aide tout type de fumeur, i.e. y compris les gros fumeurs, à arrêter de fumer, à diminuer ou à éviter de rechuter en cas d'arrêt.

Avantageusement, l'administration de la composition selon l'invention comprenant en outre des protéines extraites du cannabis permet de traiter conjointement la dépendance ou l'addiction au tabac et au cannabis.

Selon un mode de réalisation, la composition selon la présente invention est administrée par injection sous-cutanée. Selon ce mode de réalisation, la composition pharmaceutique est présentée sous une forme propre à une administration sous cutanée.

Selon un mode de réalisation, la composition pharmaceutique est présentée sous une forme de dosage de 0,03 mL à 10 mL, de préférence de 0,1 mL à 5 mL, de préférence de 0,5 à 2 mL. La composition pharmaceutique est alors de préférence administrée en une quantité de 0,03 mL à 10 mL, de préférence de 0,1 à 5 mL, de préférence de 0,5 mL à 2 mL par injection sous-cutanée.

Selon un mode de réalisation, la composition selon la présente invention est administrée à l'aide d'un système thérapeutique transdermique adhésif contenant l'extrait de feuilles de tabac tel que défini ci-dessus. Selon ce mode de réalisation, la composition pharmaceutique est présentée sous une forme propre à une administration à l'aide d'un système thérapeutique transdermique adhésif. Avantageusement, la composition pharmaceutique est présentée sous une forme de patch.

Le patch peut se présenter sous la forme d'un patch de type réservoir à un ou plusieurs compartiments ou bien de type matriciel. La réalisation pratique des patchs sera déterminée par l'homme du métier en application de ses connaissances générales en la matière pour obtenir une administration systémique contrôlée et prolongée de l'extrait de feuilles de tabac, sur toute la période d'application du patch, par exemple pendant une durée d'environ 2h à 24h.

Le type de patch réservoir comportera un ou plusieurs réservoir(s) séparé(s) contenant le ou les principe(s) actif(s) (dont l'extrait de feuilles de tabac) en solution ou en suspension dans la matrice polymère venant au contact de la peau par l'intermédiaire d'une membrane polymérique semi-perméable permettant d'ajuster la vitesse de libération du ou des principe(s) actif(s).

Le patch de type matriciel comportera une masse polymérique à l'intérieur de laquelle le ou les principe(s) actif(s) se trouveront dissous ou dispersés dans les proportions adéquates. La libération de ces principes actifs se fait par diffusion au travers des chaînes polymères de ladite matrice.

Selon un mode de réalisation particulier de ce type de patch, l'adhésif recouvre la totalité de la surface de libération de la matrice et fait partie intégrante de cette dernière. On se trouve ainsi en présence d'un patch de type adhésif actif bien connu de l'homme du métier qui relève d'une fabrication simplifiée et qui permet des réalisations de patchs de faible épaisseur et de souplesse appropriée permettant une application confortable sur la peau du patient.

Pour assurer une bonne infusion de ces principes actifs de manière sous-cutanée, ou dans la circulation sanguine, à la dose appropriée, à la vitesse de diffusion appropriée, et pour la durée appropriée, l'homme du métier pourra aisément fixer les paramètres suivants :
- le rapport des surfaces et des volumes de chacun des compartiments du patch ;
- l'ajout éventuel d'un ou plusieurs additif(s) hydrophile(s) ;
- l'ajout éventuel d'un ou plusieurs agent(s) activateur(s) ou inhibiteur(s) de diffusion ;
- l'ajout éventuel d'un ou plusieurs agent(s) de solubilisation ;
- l'ajout éventuel d'un ou plusieurs agent(s) de stabilisation ;
- l'ajout éventuel d'un ou plusieurs promoteur(s) d'absorption, et ;
- de façon plus générale, tout type d'additifs bien connus de l'homme du métier permettant de bien contrôler les flux et la stabilité de l'extrait de feuilles de tabac.

L'homme du métier saura quelle technique utiliser pour fixer les paramètres listés ci-dessus en fonction de la solubilité et de la stabilité désirée.

Les différents paramètres de fabrication du patch seront aisément adaptés l'homme du métier pour arriver au dosage souhaité.

De manière en soi connue, un tel patch comprend un film protecteur retirable qui est destiné à préserver la face adhésive à appliquer sur la peau après la fabrication du patch et durant toute sa durée de stockage. De manière en soi connue, l'homme du métier aura par exemple recours à des films polyesters dont l'une des faces peut être traitée par des silicones anti-adhérentes.

Selon un autre mode de réalisation, la composition selon la présente invention est administrée à l'aide d'un système de pulvérisation ou de vaporisation contenant l'extrait de feuilles de tabac tel que défini ci-dessus. Selon ce mode de réalisation, la composition pharmaceutique est présentée sous une forme propre à une administration par pulvérisation ou par vaporisation. Avantageusement, la composition pharmaceutique est présentée sous une forme de pulvérisateur, de vaporisateur, ou de spray.

Le pulvérisateur, le vaporisateur, ou le spray, peut se présenter sous la forme d'un pulvérisateur, le vaporisateur, ou le spray présentant un réservoir à un ou plusieurs compartiments. La réalisation pratique des pulvérisateurs, vaporisateurs, ou sprays sera déterminée par l'homme du métier en application de ses connaissances générales en la matière pour obtenir une administration systémique contrôlée et prolongée de l'extrait de feuilles de tabac.

Le pulvérisateur, le vaporisateur, ou le spray comportera un ou plusieurs réservoir(s) séparé(s) contenant le ou les principe(s) actif(s) (dont l'extrait de feuilles de tabac) en solution ou en suspension. Le ou les principe(s) actif(s) sont alors administrés par pulvérisation ou vaporisation sur la zone du corps à traiter. La pulvérisation ou vaporisation peut par exemple être réalisée sur la peau. Le ou les principe(s) actif(s) peuvent être administrés par pulvérisation ou vaporisation dans les muqueuses.

Pour assurer une bonne infusion de ces principes actifs de manière sous-cutanée, ou dans la circulation sanguine, à la dose appropriée, à la vitesse de diffusion appropriée, et pour la durée appropriée, l'homme du métier pourra aisément fixer les paramètres suivants :
- le rapport des surfaces et des volumes de chacun des compartiments ou réservoir du pulvérisateur, vaporisateur, ou spray ;
- l'ajout éventuel d'un ou plusieurs additif(s) hydrophile(s) ;
- l'ajout éventuel d'un ou plusieurs agent(s) activateur(s) ou inhibiteur(s) de diffusion ;
- l'ajout éventuel d'un ou plusieurs agent(s) de solubilisation ;
- l'ajout éventuel d'un ou plusieurs agent(s) de stabilisation ;
- l'ajout éventuel d'un ou plusieurs promoteur(s) d'absorption, et ;
- de façon plus générale, tout type d'additifs bien connus de l'homme du métier permettant de bien contrôler les flux et la stabilité de l'extrait de feuilles de tabac.

L'homme du métier saura quelle technique utiliser pour fixer les paramètres listés ci-dessus en fonction de la solubilité et de la stabilité désirée.

Les différents paramètres de fabrication du pulvérisateur, du vaporisateur, ou du spray seront aisément adaptés l'homme du métier pour arriver au dosage souhaité.

Selon un premier mode de réalisation, la composition pharmaceutique est présentée sous une forme destinée à être administrée une seule fois. Selon ce mode de réalisation, la composition pharmaceutique est administrée une seule fois permettant ainsi une diminution voire une suppression des renforcements positifs du tabac et notamment l'envie de fumer.

Selon un autre mode de réalisation, la composition pharmaceutique est présentée sous une forme destinée à être administrée plusieurs fois, de préférence deux ou trois fois. Selon ce mode de réalisation, la composition pharmaceutique est de préférence administrée à J0 et J10 ou à J0, à J10 et à J30 permettant ainsi une diminution voire une suppression des renforcements positifs du tabac et notamment l'envie de fumer. Dans ce mode de réalisation, la dose injectée la deuxième fois, et éventuellement la troisième fois, est identique à la dose injectée la première fois.

Selon un mode de réalisation particulier, la composition pharmaceutique selon la présente invention peut être administrée avec une composition comprenant un actif agissant sur le renforcement négatif, tel qu'un substitut nicotinique, la varenecline, le bupropion ou leurs mélanges. Selon ce mode de réalisation, plusieurs modes d'administration particuliers peuvent être envisagés :
(i) une administration préalable à la tentative d'arrêt de fumer de la composition pharmaceutique selon l'invention afin de diminuer la consommation de cigarettes, suivie d'une administration de l'actif agissant sur le renforcement négatif afin de limiter les effets du renforcement négatif, et notamment la sensation de manque ;
(ii) une administration concomitante de la composition pharmaceutique selon l'invention et d'un actif agissant sur le renforcement négatif au moment de la tentative d'arrêt de fumer, dans le but d'un arrêt immédiat ;
(iii) une administration au moment de la tentative d'arrêt de fumer d'un actif agissant sur le renforcement négatif, par exemple pendant 12 semaines après la tentative d'arrêt, suivie d'une administration de la composition pharmaceutique selon l'invention, par exemple pendant 2 semaines après la fin de l'administration de l'actif agissant sur le renforcement négatif, dans le but de prévenir les rechutes.

### Préparation de l'extrait de feuilles de tabac

Dans un quatrième aspect, la présente invention concerne la préparation d'un extrait de feuilles de tabac selon la présente invention.

Avantageusement, le procédé de préparation de l'extrait de feuilles de tabac selon la présente invention comprend les étapes suivantes :
a. Séchage de feuilles de tabac,
b. Broyage de feuilles de tabac séchées pour obtenir un broyat de feuilles de tabac séchées,
c. Extraction du broyat de feuilles de tabac séchées sous agitation mécanique avec un solvant, par exemple un solvant aqueux, de préférence une solution tampon aqueuse à pH compris entre 6,0 et 8,5,
d. Séparation des résidus solides de la solution de l'extrait du broyat de feuilles de tabac séchées par filtration ou centrifugation afin d'obtenir une solution d'extrait de broyat de feuilles de tabac séchées sans résidu solide,
e. Diafiltration à volume constant de la solution obtenue à l'étape d avec un solvant, de préférence un solvant aqueux, en une quantité allant de 2 à 12 fois en volume, de préférence 3 à 10 fois en volume, de préférence de 4 à 8 fois en volume, de préférence 6 fois en volume, par rapport au volume de l'extrait, et avec une membrane ayant un seuil de coupure à 10 kDa,
f. Eventuellement lyophilisation de la solution protéique obtenue à l'étape e.

Selon un mode de réalisation particulier, le séchage de l'étape a est réalisé à l'air libre, dit naturel. Dans ce cas, le séchage est réalisé en présence de l'air libre naturel, à l'extérieur ou à l'intérieur. Le séchage peut être réalisé dans un local ouvert ou fermé, couvert ou non. Un séchage à l'air libre peut par exemple être réalisé en séchoir naturel. Dans ce cas, les feuilles de tabac fraichement récoltées ou préséchées sont laissées à sécher de manière naturelle sous l'effet de l'air libre. Les feuilles de tabac peuvent par exemple être suspendues dans des hangars ventilés non chauffés. Les feuilles de tabac peuvent être par exemple laissées à sécher naturellement jusqu'à ce qu'elles prennent une couleur brune. A ce stade, il n'y a pratiquement plus de sucre dans la feuille. Avantageusement, le séchage est réalisé pour une durée adaptée aux conditions climatiques locales, de préférence pour une durée minimum d'un mois. A titre d'exemple, le séchage peut être réalisé entre septembre et décembre pour les récoltes du centre de la France. Les feuilles de tabac peuvent être retournées une ou plusieurs fois durant le séchage, de manière à permettre un séchage homogène et éviter la formation de condensation ou le pourrissement ou la dégradation des feuilles. La méthode de séchage à l'air libre peut être utilisée par exemple pour le tabac de variété Burley.Selon un mode de réalisation particulier, le séchage de l'étape a est réalisé en séchoir naturel.

Selon un autre mode de réalisation particulier, le séchage de l'étape a est réalisé en séchoir chauffant. Dans ce cas, le séchage peut être réalisé dans un hangar ou un local chauffé à une température adaptée.

Selon un autre mode de réalisation particulier, le séchage de l'étape a est réalisé par séchage au four. Dans ce cas, le séchage peut être réalisé dans un local ou un hangar chauffé à une température adaptée. La chaleur peut être introduite dans le local ou le hangar par des conduits reliés à une chaudière extérieure. Ce chauffage contrôlé permet d'obtenir des feuilles d'une couleur jaune-orange. Ces feuilles contiennent alors un fort taux de sucre. Le tabac de Virginie peut par exemple être séché selon cette méthode.

Selon un autre mode de réalisation particulier, le séchage de l'étape a est réalisé par séchage au soleil. Dans ce cas, les feuilles de tabac peuvent être étendues sur des claies et exposées au soleil pendant 12 à 30 jours. Sous la chaleur et le rayonnement directs du soleil, les feuilles prennent une couleur jaune ou orange et gardent un fort taux de sucre. Le tabac oriental est généralement séché selon cette méthode.

Selon un autre mode de réalisation particulier, le séchage de l'étape a est réalisé par séchage au feu. Dans ce cas, on peut brûler du petit bois sous les feuilles de tabac, qui sèchent en s'imprégnant d'un arôme « fumé ».

De manière avantageuse le séchage de l'étape a favorisé la dégradation des protéines de tabac de haute masse moléculaire, telle que la RuBisCO, par exemple par des mécanismes d'hydrolyse.

Les étapes c à e correspondent aux étapes d'extraction, de filtration et de diafiltration du type de celle décrites dans le brevet US 5,770,698 (colonne 6, ligne 47 à colonne 7, ligne 7) et la demande de brevet US 2009/0162403 (paragraphes [0032] à [0040]).

De préférence, l'extraction à l'étape c est réalisée à une température comprise entre 4 et 20 °C, de préférence entre 4 et 10 °C.

De préférence, l'extraction à l'étape c est réalisée pendant 12 à 36 h, de préférence 22 à 26h, de préférence 24h.

Selon un mode de réalisation particulier, le solvant utilisé à l'étape c est un solvant aqueux. De préférence, le solvant aqueux utilisé à l'étape c est une solution tampon aqueuse de bicarbonate d'ammonium, de préférence à une concentration comprise entre 2 et 6 g/L, de préférence de 4 g/L.

Bien entendu, d'autres types de solvants peuvent être utilisés pour réaliser l'extraction de l'étape c. L'extraction de l'étape c peut être réalisée avec un solvant organique ou inorganique, ou avec un mélange de solvants organiques et/ou inorganiques.

Pour assurer une bonne efficacité et un bon rendement d'extraction, et pour obtenir un extrait de bonne qualité présentant les propriétés de pureté et la composition désirées, l'homme du métier saura aisément sélectionner le ou les solvants appropriés selon les critères suivants :
- la polarité du solvant ou du mélange de solvants (polaire ou apolaire) ;
- l'état physique du solvant ou du mélange de solvants (par exemple liquide, solide, supercritique, ou gazeux) ;
- la nature chimique du solvant ou du mélange de solvants (par exemple organique ou inorganique) ;
- la charge du solvant ou du mélange de solvants (par exemple ionique ou non ionique) ;
- l'origine du solvant ou du mélange de solvants ;
- la miscibilité du solvant ou du mélange de solvants ;
- la solubilité du solvant ou du mélange de solvants.

L'homme du métier saura quelle technique utiliser pour sélectionner le ou les solvant(s) correspondant à ces critères, en fonction du rendement d'extraction, de la pureté et de la composition de l'extrait désirés.

De préférence, l'extraction à l'étape c est réalisée en mettant en suspension le broyat de feuilles de tabac séchées dans une solution tampon, de préférence à une concentration en broyat de feuilles de tabac séchées dans la solution tampon de 30 à 70 g/L, de préférence de 40 à 60 g/L, et plus préférentiellement de 50 g/L. Les résidus solides en suspension sont ensuite éliminés par filtration, par exemple par filtration sur Büchner (étape d), afin d'obtenir un broyat de feuilles de tabac séchées sans résidu solide.

De préférence, le solvant aqueux utilisé à l'étape d est de l'eau PPI (eau pour préparation injectable).

De manière avantageuse, l'étape e permet d'éliminer plus de 99 % des molécules de masse moléculaire inférieure à 10 kDa et notamment des acides aminés libres, des protéines et peptides de masse moléculaire inférieure à 10 kDa et des résidus protéiques de masse moléculaire inférieure à 10 kDa issus de la dégradation des protéines de l'étape a.

De préférence, la solution protéique obtenue à l'étape e est soumise à une étape e', préalablement à l'étape f, de filtration stérilisante.

L'extrait de feuilles de tabac selon l'invention obtenu à l'issue du procédé décrit ci-dessus peut être conservé tel qu'obtenu à l'issu de l'étape d' de filtration stérilisante, ou lyophilisé tel qu'obtenu à l'issue de l'étape f.

### Préparation de composition pharmaceutique comprenant l'extrait de feuilles de tabac

Dans un cinquième aspect, la présente invention concerne la préparation de la composition pharmaceutique comprenant l'extrait de feuilles de tabac selon l'invention.

La composition pharmaceutique peut être préparée selon un procédé comprenant les étapes suivantes :
a. Préparation d'un extrait de feuilles de tabac tel que défini ci-dessus,
b. Ajustement de la concentration protéique de sorte à obtenir une concentration en protéine allant de 100 à 200 µg/mL,
c. Addition du ou des excipients pharmaceutiquement acceptables, et
d. Eventuellement addition à cet extrait d'adjuvant(s), de préférence addition de mannitol.

L'extrait de feuilles de tabac utilisé à l'étape b peut être soit celui directement obtenu après l'étape de diafiltration sur membrane ayant un seuil de coupure à 10 kDa, soit celui obtenu après l'étape de diafiltration stérilisante, soit l'extrait de feuilles de tabac lyophilisé puis reconstitué par exemple dans du sérum physiologique ou de l'eau.

Avantageusement, l'ajustement de la concentration à l'étape b peut être réalisé :
- soit par dilution avec de l'eau afin d'abaisser la concentration en protéines,
- soit par diafiltration afin d'augmenter la concentration en protéines.

Un excipient pharmaceutiquement acceptable particulièrement préféré à l'étape c est l'eau.

Les adjuvants pouvant être additionnés à l'étape d peut être notamment les agents de gonflement comme par exemple un sucre tel que le lactose, le saccharose, le tréhalose, le sorbitol, le glucose, le raffinose, le mannitol, de préférence le lactose, le saccharose, le tréhalose, le glucose, ou le mannitol, un acide aminé tel que l'arginine, la glycine, ou l'histidine, de préférence la glycine, ou des polymères de type dextrane ou polyéthylène glycol ; ou leurs mélanges.

Selon un mode de réalisation particulier, l'étape d comprend au moins d'addition de mannitol.

### Kit comprenant la composition pharmaceutique comprenant l'extrait de feuilles de tabac

Dans un sixième aspect, la présente invention concerne un kit comprenant des doses d'extrait de feuilles de tabac ou de composition pharmaceutique selon l'invention.

Selon un premier mode de réalisation, le kit comprend une ou des dose(s) d'extrait de feuilles de tabac, de préférence sous forme lyophilisée, ainsi qu'une ou des dose(s) de sérum physiologique ou d'eau PPI pour préparer la composition pharmaceutique selon l'invention juste avant l'administration.

Selon un autre mode de réalisation, le kit comprend une ou des dose(s) de composition pharmaceutique prête(s) à être administrée(s) au patient.

Le kit peut éventuellement comprendre une ou des seringue(s) afin d'administrer la composition pharmaceutique par injection sous-cutanée.

Le kit peut éventuellement comprendre un ou des patch(s) afin d'administrer la composition pharmaceutique de manière transdermique.

Les exemples qui suivent visent à illustrer la présente invention.

### EXEMPLES

### Exemple 1 : Préparation d'un extrait de feuilles de tabac selon l'invention

Des feuilles de tabac Burley sont utilisées pour préparer l'extrait.

100 g de feuilles de tabac Burley séchées par séchage naturel pendant environ trois mois (dans le centre de la France, entre septembre et décembre) sont broyés. Le broyat est mis en suspension pendant 24h à une température de 4 à 10 °C dans 1 892 g d'eau PPI à laquelle 8 g de bicarbonate d'ammonium ont été ajoutés. Les résidus solides en suspension sont ensuite éliminés par filtration sur Büchner.

Une filtration clarifiante à 0,2 µm de l'extrait est alors réalisée. L'extrait obtenu est de couleur brune. L'extrait est ensuite pesé afin de déterminer le volume d'eau PPI à utiliser lors de l'étape de diafiltration à volume constant, la masse de l'extrait liquide est alors de 1 680 g.

Une extraction des protéines de cet extrait de broyat de feuilles de tabac séchées sans résidu solide est ensuite réalisée : 10 080 g d'eau PPI sont alors ajoutés à l'extrait de broyat de feuilles de tabac séchées sans résidu solide. Les 11 760 g de solution ainsi obtenus sont diafiltrés à volume constant contre 6 fois le volume avec un seuil de coupure à 10 kDa (MerckMillipore) jusqu'à ramener la masse du rétentat jusqu'à 1 680 g.

Le rétentat obtenu après la diafiltration constitue un mélange protéique dans lequel les protéines de masse moléculaire inférieure à 10 kDa sont décelées avec une concentration de 99 % inférieure à leur concentration initiale. La couleur du rétentat obtenu est située entre B2 et B3 selon l'échelle de mesure EUROPEAN PHARMACOPOEIA 5.0, 2.2.2, Degree of coloration of liquids.

Ce diafiltrat peut être lyophilisé, après ajout de mannitol par exemple, par exemple à l'aide d'un lyophilisateur SMH 150, pour obtenir un extrait de feuilles de tabac lyophilisé.

### Exemple 2 : Détermination de la composition protéique de l'extrait de feuilles de tabac préparé selon l'exemple 1

Les protéines de l'extrait de feuilles de tabac de l'exemple 1 sont préalablement séparées sur un gel de polyacrylamide. Pour cela, l'extrait à analyser est ajouté à un gel NuPage^{®} Bis-Tris (4-12 %) et l'électrophorèse est effectuée à 200 V pendant 35 min, avant d'être mis au contact de 20 mL d'Instant Blue pendant 1h puis rinsé à l'eau pendant une nuit. Les bandes de gel de polyacrylamide sélectionnées sont excisées (6 bandes), puis décolorées avec un tampon NH₄CO₃ 50 mM/CH₃CN (50/50). Les ponts disulfures sont ensuite réduits avec une solution de dithiothréitol 10 mM/NH₄CO₃ 50 mM pendant 40 min à 55 °C. Les cystéines réduites sont ensuite alkylées avec une solution d'iodoacétamide 100 mM/NH₄CO₃ 50 mM pendant 30 min à température ambiante et dans l'obscurité.

La solution protéique ainsi obtenue est digérée grâce à une enzyme afin d'obtenir des fragments de protéines ou de peptides : la digestion est réalisée avec de la trypsine (trypsine V5111 de Promega) en quantité adaptée à la coloration des bandes de gel de polyacrylamide dans une solution de NH₄CO₃ 50 mM pendant une nuit à 37 °C.

Les peptides du digestat peptidique sont alors séparés par nanochrommatographie liquide (appareil U3000 nanoLC system de ThermoFischer Scientific) avec préconcentration sur C18 PepMap micro-precolumn (5 µm; 100 Å; 300 µm x 5 mm; ThermoFisher Scientific) puis élution sur C18 PepMap nanocolumn (3 µm; 100 Å; 75 µm x 250 mm; ThermoFisher Scientific) en mode gradient linéaire ; tampon A 0.1 % HCOOH dans H₂O/CH₃CN (95/5), tampon B 0.1 % HCOOH dans H₂O/CH₃CN (20/80), Gradient 0 à 60 % B en 60 min, débit de 300 µL/min).

Les peptides sont ensuite analysés par spectrométrie de masse sur un instrument LTQ Velos (Dual Pressure Linear Ion Trap; ThermoFisher Scientific) équipé d'une source nanospray (ThermoFisher Scientific) et couplé à l'appareil U3000 nanoLC. Les données sont acquises avec le logiciel Excalibur 2.1 (ThermoFisher Scientific) en mode positif par cycle de un scan MS de m/z 400 à 1600 en mode « Resolution Enhanced » suivie de scans MSMS en mode « Resolution Normal » sur les 20 ions MS les plus intenses (charge 2 et plus) en mode CID sous Hélium avec une énergie de collision de 35 eV. Les ions MS précédemment fragmentés sont exclus dynamiquement pendant 30 s avec une tolérance en masse de 50 mmu.

Les masses des peptides et de leurs fragments sont comparées aux données existantes dans les banques de données afin de les identifier. Pour cela, les données MS et MSMS sont traitées avec le logiciel ProteomeDiscoverer 1.4 selon l'algorithme de recherche MASCOT (Version 2.4) et la banque de données UniprotKB/Swiss-Prot (release April 2015) réduite à l'espèce TOBAC est interrogée.

L'identification des protéines est validée selon la valeur de confiance p inférieure à 0,05. Seules les protéines identifiées avec au moins 2 peptides de confiance maximale sont retenues.

Les scores de chaque protéine ainsi identifiée ne permettent qu'un classement relatif des protéines entre elles, ce classement étant corrélé aux concentrations de ces protéines. Les valeurs absolues de ces scores dépendent des conditions opératoires, de la prise d'essai, des quantités déposées.

La liste des 12 protéines ainsi identifiées et classées par ordre décroissant de leurs scores (de la plus présente à la moins présente) est la suivante :
- P36401 ; E13H_TOBAC ; Glucan endo-1,3-beta-glucosidase, acidic isoform PR-Q' (EC 3.2.1.39) ((1->3)-beta-glucan endohydrolase) ((1->3)-beta-glucanase) (Beta-1,3-endoglucanase) (PR-35) ; MM : 36995 Da
- P17513 ; CHIP_TOBAC ; Acidic endochitinase P (EC 3.2.1.14) (Pathogenesis-related protein P) (PR-P); MM: 27469 Da
- P17514 ; CHIQ_TOBAC ; Acidic endochitinase Q (EC 3.2.1.14) (Pathogenesis-related protein Q) (PR-Q); MM: 27633 Da
- P27666 ; E13F_TOBAC ; Glucan endo-1,3-beta-glucosidase, basic vacuolar isoform GLB (EC 3.2.1.39) ((1->3)-beta-glucan endohydrolase) ((1->3)-beta-glucanase) (Beta-1,3-endoglucanase, basic) (Glucanase GLB); MM: 40443 Da
- P14170 ; OSMO_TOBAC ; Osmotin; MM: 26681 Da
- P24091 ; CHI2_TOBAC ; Endochitinase B (CHN-B) (EC 3.2.1.14); MM: 34721 Da
- P11965 ; PERX_TOBAC ; Lignin-forming anionic peroxidase (EC 1.11.1.7) (TOPA); MM: 34674 Da
- P13046; PRR1_TOBAC ; Pathogenesis-related protein R major form (Thaumatin-like protein E22); MM: 24667 Da
- P29062 ; PR4A_TOBAC ; Pathogenesis-related protein PR-4A; MM: 16221 Da
- Q03199 ; IPIB_TOBAC ; Proteinase inhibitor I-B (PI-IB) (Inhibitor of microbial serine proteinases major isoform); MM: 11916 Da
- Q03198 ; IPIA_TOBAC ; Proteinase inhibitor I-A (PI-IA) (Inhibitor of microbial serine proteinases minor isoform); MM: 11880 Da
- P29063 ; PR4B_TOBAC ; Pathogenesis-related protein PR-4B ; MM : 16235 Da

Le procédé de préparation de l'extrait de feuilles de tabac permet donc de concentrer certaines protéines et d'en éliminer d'autres. Ainsi, parmi les 759 protéines de l'espèce *Nicotiana tabacum* listées dans Swissprot, uniquement 6 familles et 12 protéines sont très majoritairement présentes dans l'extrait de feuilles de tabac préparé selon l'exemple 1. Les 12 protéines qui sont très majoritairement présentes dans l'extrait de feuilles de tabac préparé selon l'exemple 1 présentent une masse moléculaire comprise entre 10 et 50 kDa.

### Exemple 3 : détermination de la teneur en protéines

La teneur en protéines dans l'extrait de feuilles de tabac selon l'exemple 1 (avant et après l'étape de diafiltration) a été déterminée selon la méthode de Bradford.

L'extrait de l'exemple 1 est dissout dans du sérum physiologique.

Trois solutions standard de protéine ont été préparées : 50 µL d'une solution d'albumine de sérum bovin à 2mg/mL (Sigma Aldrich, référence P0834) sont dilués 20 fois avec 950 µL de sérum physiologique afin d'obtenir des solutions standardisées à exactement 100 µg/mL.

Chaque tube testé comprend 80 µl d'échantillon de protéine et 920 µL de réactif de Bradford (Sigma Aldrich référence B6916). Après addition du réactif de Bradford dans chaque tube, les tubes sont agités doucement de sorte à observer un vortex, puis incubés pendant 5 min à température ambiante. Les échantillons sont transférés dans des cuvettes de 1,5 mL et leur absorbance est mesurée à 595 nm pendant 1h.

La concentration en protéine a été déterminée par comparaison avec les solutions standards de protéine préparées.

Les quantités de protéines mesurées sont de :
- avant diafiltration : 295,45 +/- 9,91 µg/mL
- après diafiltration : 160,61 +/- 1,31 pg/mL.

Le profil électrophorétique de l'extrait de feuilles de tabac selon l'exemple 1 a été déterminé après l'étape de diafiltration. Des quantités de 2, 5, 10 and 15 µg de protéines ont été déposées sur un gel NuPage.

Le gel Nupage 4 % -12 % a été préparé pendant 35 minutes puis mis dans 20 ml de Bleu de Commassie pendant 1 h et rincé avec de l'eau pendant une nuit. Le gel a été analysé en utilisant le système ChemiDoc^{™} XRS de Biorad avec le logiciel Image Lab^{™}.

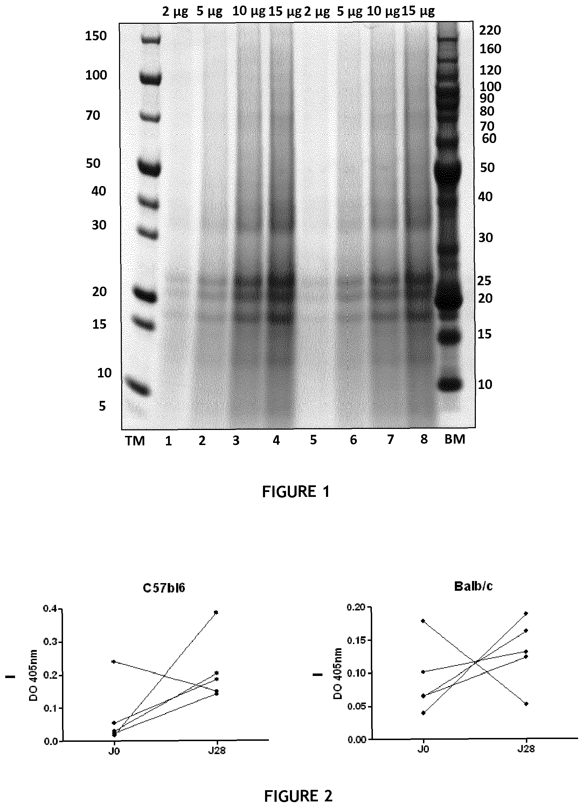

Trois bandes principales sont détectées aux alentours de 15 kDa et 30 kDa (Fig. 1). Deux autres bandes sont également perceptibles à environ 40 kDa.

Les résultats montrent que la quantité de protéines de masse moléculaire supérieure à 100 kDa détectée est faible comparé à la quantité de protéines de masse moléculaire comprise entre 10 et 50 kDa, dans cet extrait de feuilles de tabac.

### Exemple 4 : Influence du volume de diafiltration sur l'élimination des molécules de masse moléculaire inférieure à 10 kDa dans un extrait de feuilles de tabac selon l'invention

L'élimination des molécules de poids moléculaire inférieur à 10 kDa dans l'extrait de feuilles de tabac selon l'exemple 1 a été suivie lors de l'étape de diafiltration selon le volume de diafiltration utilisé. Le suivi est effectué par dosage par chromatographie en phase gazeuse d'un traceur analytique (nicotine) de masse moléculaire inférieure à 10 kDa.

Les résultats sont reportés dans le tableau suivant :

| Prélèvements en fonction du volume de diafiltration | Aire de pics du traceur analytique | Pourcentage de traceur analytique restant |
|---|---|---|
| Avant diafiltration | 54913 | 100 % |
| Diafiltration x3 volume | 2630 | 4,79 % |
| Diafiltration x4 volume | 809 | 1,47 % |
| Diafiltration x5 volume | 247 | 0,045 % |
| Diafiltration x6 volume | NQ | NQ |

L'élimination des molécules de poids moléculaire inférieur à 10 kDa est de plus en plus efficace lorsque le volume de diafiltration augmente et est totale lorsque ce volume de diafiltration est de 6 fois le volume initial.

### Exemple 5 : activité biologique de l'extrait de l'exemple 1 in vitro sur des cellules mononucléées humaines et in vivo sur des souris

### Matériels et méthodes :

### - Produits utilisés

PMA/Ionomycin (référence P8139/I0634 de Sigma-Aldrich)
BSA (référence A7020 de Sigma Aldrich)
PBS (référence H15-002 de PAA Laboratories)
Tween 20 (référence P1379 de Sigma Aldrich)
Goat anti-mouse IgG (g chain specific)- Alkaline Phosphatase (référence 1030-04 de SouthemBiotech)
Biotin-anti mouse IgE (référence BLE406904 de Biolegend)
Streptavidin - Alkaline Phosphatase (Southem Biotech ref7100-04)

### - Préparation et mode de stimulation des cellules

Des cellules mononucléées du sang périphérique de différents donneurs sains ont été isolées par centrifugation sur un gradient de densité (milieu de séparation : LMS 1077 PAA Laboratories). Les cellules mononucléées obtenues ont été stimulées avec l'extrait selon l'exemple 1 à différentes dilutions pendant 24h ou 48h. Les surnageants ont été prélevés puis stockés à -80°C après 24h ou 48h de stimulation pour l'analyse du profil cytokinique par la technique Luminex. Après 24h de stimulation les cellules ont également été prélevées pour l'analyse phénotypique par cytométrie de flux.

### - Analyse phénotypique par cytométrie

Les cellules ont été marquées dans un premier temps par un marqueur de viabilité (Dye eFluor 450 (65-0863-14 eBioscience) puis dans un deuxième temps par des anticorps couplés directement à des fluorochromes pour la détection de différents marqueurs membranaires. Les populations de lymphocytes T et de lymphocytes B ont été identifiées avec les marqueurs CD3 et CD19. Les cellules NK ont été détectées soient avec le marqueur NKp46 ou la combinaison des marqueurs CD56 et CD16 ou via l'expression de CD56 en l'absence de CD3. A partir de ces populations cellulaires, des « gates » ont été effectuées pour analyser différents marqueurs d'activation (CD69, CD25, HLA-DR).

La liste des anticorps utilisés pour l'analyse phénotypique par cytométrie est donnée dans le tableau suivant :

| | Clone | Référence et fournisseur |
|---|---|---|
| Anti-CD45-Krome Orange | J.33 | A96416 ; Beckman Coulter |
| Anti-CD56 APC | B159 | 555518 ; Becton Dickinson |
| Anti-CD25PC7 | M-A251 | 557741 ; Becton Dickinson |
| Anti-CD25 PC5 | B1.49.9 | IM2646 ; Beckman Coulter |
| Anti-CD69 FITC | FN50 | 557049 ; Becton Dickinson |
| Anti-CD69 PE | L78 | 341652 ; Becton Dickinson |
| Anti-CD3 ECD | UCHT1 | A07748 ; Beckman Coulter |
| Anti-KpP46 APC | 9E2/NKp46 | 558051 ; Becton Dickinson |
| Anti-CD19 FITC | HIB19 | 11-0199-42 ; eBioscience |
| Anti-HLA-DR PE | G46-6 | 347401 ; Becton Dickinson |

Des contrôles isotypes ont été utilisés dans chaque expérience et une matrice de compensation réalisée pour cette analyse phénotypique multiparamétrique.
Les échantillons marqués ont été analysés sur un cytomètre Navios (Beckman Coulter 10 couleurs) avec une acquisition d'un minimum de 100 000 évènements par tube. Les données acquises ont ensuite été analysées sur le logiciel Kaluza.

### - Analyse du profil de sécrétion cytokinique par Luminex et ELISA

Cette technologie basée sur des principes de technique ELISA et de cytométrie permet le dosage de plusieurs analytes simultanément.

Des microbilles possédant un code couleur unique sont couplées à des anticorps de capture spécifique pour chaque analyte. Après une incubation avec les échantillons à doser, des anticorps de détection couplés à un fluorochrome vont permettre l'analyse par un système optique de deux lasers (Bio-plex 200 system Bio-rad). Le premier laser permet l'identification de la microbille et le second permet de quantifier les anticorps de détection couplés au fluorochrome. 17 analytes peuvent être dosés avec le kit Bio-Plex Pro Human Cytokine Grp1 panel 17 plex (Bio-rad) sur les échantillons prélevés au cours des différentes expériences de stimulations réalisées sur les cellules. Les cellules stimulées par le milieu de culture sans l'extrait de feuilles de tabac selon l'exemple 1 permettent d'obtenir le taux basal pour chaque cytokine.

L'ELISA IL-8 repose sur une technique sandwich immunoenzymatique classique. Les trousses commerciales utilisées ont été achetées chez Eurobio-Diaclone (Besançon).

### - Immunisation des souris avec l'extrait de feuilles de tabac selon l'exemple 1

Des souris femelles de 7 semaines C57BL/6 et Balb/c ont été achetées chez Charles River (L'Arbresles, France). Les souris ont été immunisées à J0 et J21 par voie intrapéritonéale avec 200 µL de l'extrait de feuilles de tabac selon l'exemple 1 lyophilisé puis reconstitué dans 5 mL de sérum physiologique. Du sang de souris a été prélevé chez toutes les souris avant et après immunisation. Après centrifugation, le sérum a été congelé à -20°C.

### - ELISA IgG et IgE

Pour l'expérience ELISA, un flacon du produit a été reconstitué avec 2,5 mL de tampon carbonate-bicarbonate (0.1M, pH 9). Des plaques Nunc Maxisorb 96W ont été incubées avec 100 µL de l'extrait de feuilles de tabac lyophilisé selon l'exemple 1 puis reconstitué, et laissées incuber 18h. Après lavage des plaques en tampon PBS-Tween 0,05 %, les plaques sont saturées avec 200 µL de tampon PBS-BSA 1 % pendant une heure à température ambiante. Après lavage des plaques en tampon PBS-Tween 0,05 %, 100 µL de sérum des souris, dilué au 1/20 dans du PBS-BSA 1 % sont incubés pendant 2 heures à température ambiante.

### Elisa IgG :

Après lavage en tampon PBS-Tween 20, 100 µL d'un anticorps secondaire de chèvre anti-IgG de souris couplé à la phosphatase alcaline (Southem Biotechnology réf 1030-04) et dilué dans du PBS-BSA 1 % est incubé pendant 1 heure à température. Après des lavages en PBS-Tween 0.05 %, 100 µL du substrat p-Nitrophenyl phosphate (pNPP de Sigma) de la phosphatase alcaline est ajouté et laissé 15 min à température ambiante à l'obscurité. La réaction est ensuite arrêtée en ajoutant 50 µL de NaOH 3M et les densités optiques (DO) sont lues à 405 nm.

### Elisa IgE

Après lavage en tampon PBS-Tween 20, 100 µL d'un anticorps secondaire de rat anti-IgE de souris couplé à la biotine (Biolegend Ref BLE406904) et dilué dans du PBS-BSA 1 % est incubé pendant 1 heure à température ambiante. Après des lavages en PBS-Tween 0,05 %, 100 µL de streptavidine couplée à la phosphatase alcaline (Southern Biotechnology Ref 7100-04) est ajouté et incubée pendant 1h à température ambiante (dilution 112000^{ème} dans PBS-BSA 1 % selon les recommandations du fournisseur). Après de nouveaux lavages en PBS-Tween 0,05 %, 100 µL du substrat pNPP de la phosphatase alcaline est ajouté et laissé 15 min à température ambiante à l'obscurité. La réaction est ensuite arrêtée en ajoutant 50 µL de NaOH 3M et les densités optiques (DO) sont lues à 405 nm.

### Résultats :

### - L'extrait de feuilles de tabac selon l'exemple 1 entraine une réponse humorale de type IgG spécifique chez des souris.

Après 2 immunisations de souris avec l'extrait de feuille de tabac lyophilisé selon l'exemple 1 puis reconstitué dans 5 mL de sérum physiologique, une induction d'IgG a été observée contre des composants du produit. Ainsi, 4/5 souris C57BL/6 et 4/5 souris Balb/c ont présenté une augmentation de leur IgG contre l'extrait de feuilles de tabac à J28, soit 7 jours après la 2^{ème} vaccination (Fig. 2). La seule souris dans chaque groupe n'ayant pas répondu avait des taux d'anticorps de type IgG à l'état basal. Les titres d'anticorps semblent plus faibles chez les souris Balb/c que chez les souris C57BL/6 (Fig. 2).

Par contre, aucune réponse humorale de type IgE après 2 vaccinations des souris avec l'extrait de feuilles de tabac n'a été détectée. Les densités optiques (DO) étaient inférieures à 0,05 dans tous les dosages réalisés sur les différents sérums.

### - L'extrait de feuilles de tabac active préférentiellement les cellules Natural Killer du sang périphérique

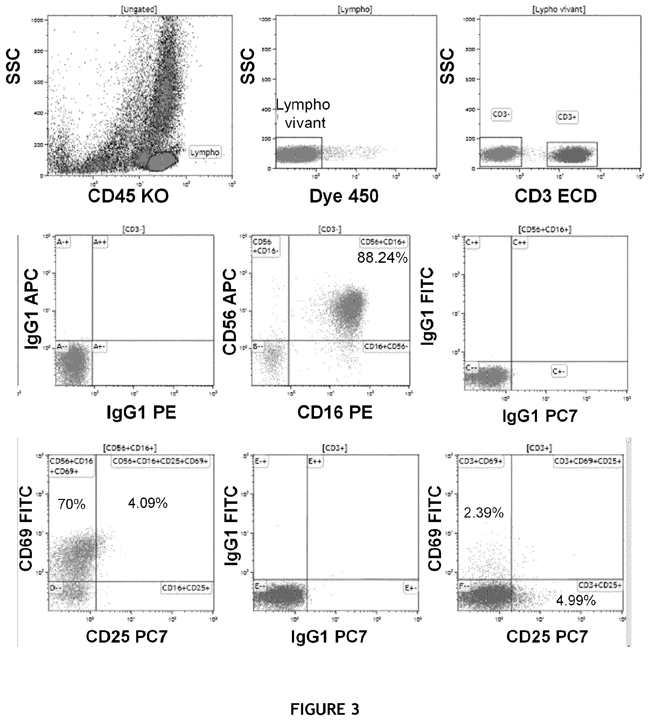

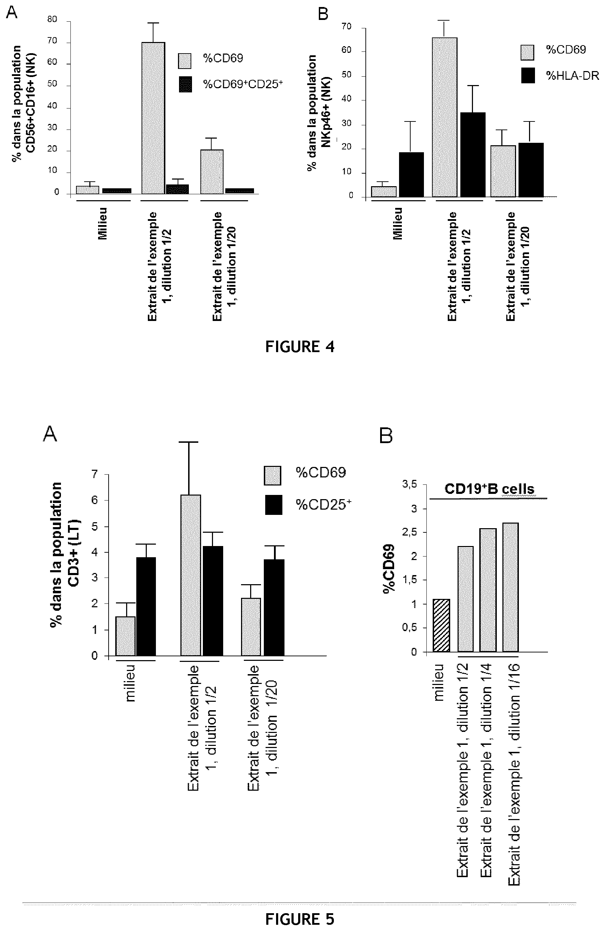

Après isolement des cellules mononucléées du sang périphérique par gradient de densité (Ficoll), différentes populations cellulaires (lymphocytes T et B, cellules NK...) peuvent être caractérisées (Fig. 3). Ainsi les cellules NK ont été identifiées par les marqueurs CD56 et CD16 dans la population CD3 négative ou par le marqueur NKp46. Après incubation des cellules mononucléées avec l'extrait de feuilles de tabac selon l'exemple 1, une augmentation d'expression des marqueurs d'activation CD69, CD25 et HLA-DR a été observée sur les cellules NK aussi bien identifiée par les marqueurs CDI6+CD56+ (CD3-) (Fig. 4A) que par le marqueur NKp46 (Fig. 4B). Les résultats sont plus marqués avec l'extrait de feuilles de tabac dilué au ½ et pour l'expression du CD69. Ainsi plus de 60 % des cellules NK expriment le marqueur CD69, 24 heures après la mise en contact avec l'extrait de feuilles de tabac (Fig. 4A-B). Au repos, moins de 5 % des cellules NK du sang expriment CD69.

L'effet de l'extrait de feuilles de tabac sur l'activation des lymphocytes du sang périphérique est beaucoup plus faible. Ainsi, au repos 2 % des lymphocytes T expriment CD69 et cette expression augmente à 6 % après 24 heures de culture avec l'extrait de feuilles de tabac (Fig. 5A). L'expression du CD25 n'est pas augmentée sur les lymphocytes T sensibilisés avec l'extrait de feuilles de tabac (Fig. 5A). De même, sur les lymphocytes B du sang périphérique, l'extrait de feuilles de tabac n'a pas d'activité significative, car moins de 3 % des lymphocytes B expriment CD69 après 24 heures de co-culture avec cet extrait (Fig. 5B).

### - Profil de cytokines induit par l'extrait de feuilles de tabac selon l'exemple 1 sur les cellules mononucléées du sang humain

Des cellules mononucléées du sang périphérique ont été mises en contact pendant 24 heures avec l'extrait de feuilles de tabac lyophilisé selon l'exemple 1 puis reconstitué avec 5 mL de PBS à différentes dilutions (1/2 ou 1/20^{ème}) et des dosages de cytokines, chimiokines et facteurs de croissance ont été réalisées par Luminex dans le surnageant.

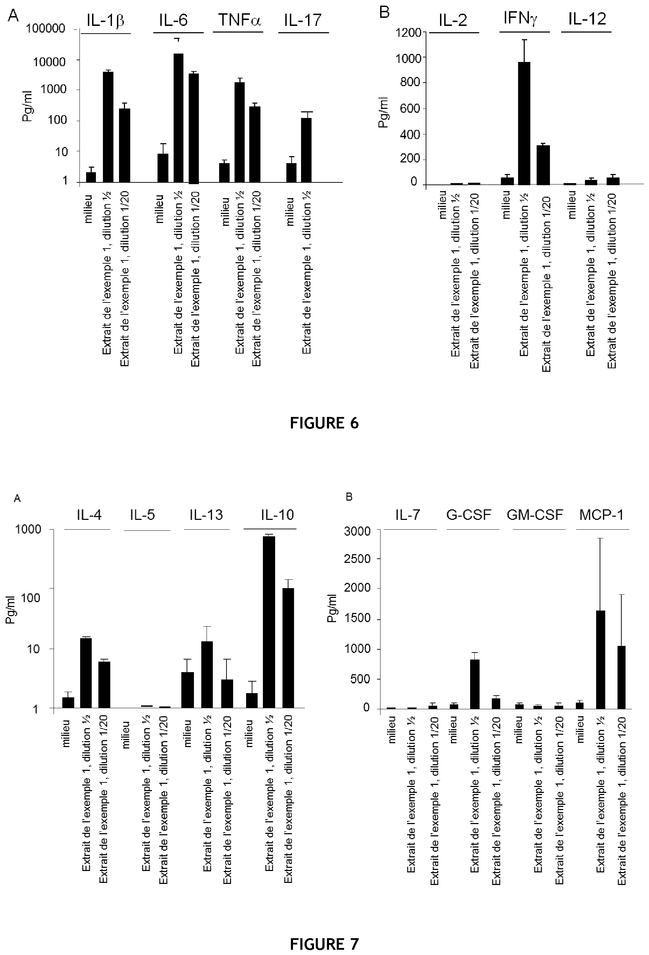

La figure 6 montre une induction de cytokines pro-inflammatoires (IL-1B, IL-6, TNFα, IL-17) importantes par l'extrait de feuilles de tabac. Ainsi les taux basaux de ces cytokines lorsque les cellules mononucléées ne sont pas mises en présence de l'extrait de feuilles de tabac sont inférieurs à 10 pg/mL (Fig. 6A).

Les concentrations des cytokines IL-1B, IL-6 et TNFα sont mesurées à plus de 1 000 pg/mL après incubation avec l'extrait de feuilles de tabac dilué au ½ (Fig. 6A). Cette nette induction de cytokines pro-inflammatoires est également observée lorsque l'extrait de feuille de tabac est dilué au 1/20^{ème} (Fig. 6A). Les résultats sont assez homogènes entre les différents donneurs testés. Parmi les cytokines TH1 (IL-2, IFNγ, IL-12) favorisant une immunité à médiation cellulaire, seule l'IFNγ est significativement stimulée par l'extrait de feuilles de tabac dilué au 1/2 et au 1/20^{ème} (Fig. 6B). L'extrait de feuilles de tabac induit de très faibles concentrations de cytokines TH2 (IL-4, IL-5, IL-13) de l'ordre de quelques pg/mL (Fig. 7A). Par contre, l'extrait de feuilles de tabac stimule la production d'IL-10 par les cellules mononucléées avec des taux de 750 pg/mL retrouvés dans le surnageant des cellules mises en contact avec l'extrait de feuilles de tabac dilué au 1/2 (Fig. 7A). L'IL-10 était initialement considérée comme une cytokine TH2 mais elle peut être produite aussi par des lymphocytes T régulateurs appelés Tr1.

Nous avons montré que l'extrait de feuilles de tabac pouvait également induire la production de facteurs de croissance hématopoïétique comme le G-CSF qui favorise l'expansion et le recrutement de neutrophiles et de chimiokines comme le MCP-1 connue pour exercer un effet chimiotactique sur les macrophages (Fig. 7B). Par contre, l'extrait de feuilles de tabac ne semble pas stimuler de façon significative d'autres facteurs de croissance comme l'IL-7 ou le GM-CSF (Fig. 7B). Le même profil de cytokines/chimiokines a été retrouvé lorsque les dosages ont été réalisés sur des surnageants recueillis 48 heures après la mise en culture avec l'extrait de feuilles de tabac.

### Conclusion

L'extrait de feuilles de tabac selon l'exemple 1 est capable d'induire une réponse humorale de type IgG spécifique dirigée contre des protéines présentes dans le produit chez deux lignées de souris de fond génétique différents.

Aucune réponse humorale de type IgE n'a été observées après deux administrations du produit chez ces mêmes souris.

*In vitro,* l'extrait de feuilles de tabac selon l'exemple 1 induit préférentiellement l'activation des cellules NK parmi les cellules mononucléées du sang périphérique. Cette activation est reflétée par l'augmentation d'expression des molécules CD69, CD25 et HLA-DR.

L'extrait de feuilles de tabac selon l'exemple 1 induit *in vitro* des cytokines pro-inflammatoires comme l'IL-1B, l'IL6, l'IL-17, l'IL-8 et du TNFα. Ces cytokines sont présentes à fortes concentrations dans le surnageant de culture prélevé 24 heures après la stimulation.

L'extrait de feuilles de tabac selon l'exemple 1 induit également de l'IFNγ, de l'IL-10, du G-CSF et du MCP-1. Par contre, cet extrait n'induit pas de façon significative la production de cytokines TH2 (IL-4, IL-5, IL-13).

### Exemple 6 : étude toxicologique d'un extrait de feuilles de tabac selon l'exemple 1

### Matériels et méthodes :

Toutes les études ci-dessous ont été réalisées avec l'extrait de feuilles de tabac selon l'exemple 1.

La voie d'administration est la voie sous-cutanée dans tous les cas. Les études toxicologiques ont été réalisées sur des rongeurs (rats Han Wistar). Les études toxicologiques ont été réalisées selon les principes de l'OCDE en matière de bonnes pratiques de laboratoire (Acceptation Mutuelle des Données (AMD) dans l'évaluation des données, 26 novembre 1997 (C(97) 186 Final) et selon les Bonnes Pratiques de Laboratoire (GLP) publiées par le Ministère français de l'Emploi et de la Solidarité (n° 2000/5bis, arrêté du 14 mars 2000, JO 23/03/2000).

### - Etude de la toxicité sous-cutanée à 14 jours

L'objectif de cette étude a été de déterminer la toxicité de l'extrait de feuilles de tabac selon l'exemple 1 dans les rats suivant l'administration sous-cutanée (une fois par semaine pendant 2 semaines).

L'étude a été menée comme suit :

| Groupe n° | Dose exprimée en protéines (µg/kg/adm) | Volume de dose (mL/kg/adm) | Concentration de dose exprimée en protéines (µg/mL) | Nombre d'animaux | |
|---|---|---|---|---|---|
| | | | | males | femelles |
| 1 | 0 | 2 | 0 | 6 | 6 |
| 2 | 11,2 | 0,4 | 28 | 6 | 6 |
| 3 | 56 | 2 | 28 | 6 | 6 |

Les résultats de l'étude ont montré que :
- aucune mortalité n'est observée quelle que soit la dose ;
- aucun signe clinique lié au traitement n'est observé et l'administration sous-cutanée est localement bien tolérée (les signes clinique et la tolérance locale ont été observés avant et après injection, et une fois par jour quand aucun traitement n'est administré) ;
- les conséquences sur le poids corporel et la consommation de nourriture (comparés aux contrôles) ne sont pas préjudiciables ;
- la leucocytémie présente des différences, entre les rongeurs traités et non traités, considérées comme significatives biologiquement mais sans effet toxique ;
- des variations ont été observées entre le groupe traité et celui non traité en ce qui concerne les concentrations en protéines et en cholestérol, mais ces variations sont restées dans ou proches de celles du groupe contrôle. Ces effets ne sont pas préjudiciables ;
- aucune modification n'a été observée pour ce qui est du poids des organes entre le groupe traité et le groupe contrôle.

L'administration sous-cutanée aux rats Wistar de l'extrait de feuille de tabac selon l'exemple 1 une fois par semaine pendant 2 semaines à des doses de 11,2 et de 56 µg de protéines/kg/adm a été donc bien tolérée.

### - Etude de la toxicité sous-cutanée pendant 4 semaines

L'objectif était de déterminer la toxicité de l'extrait de feuilles de tabac selon l'exemple 1 suivant l'administration sous-cutanée une fois par semaine à des rats Wistar et de déterminer la régression de tout signe de toxicité durant une période de 4 semaines sans traitement.

Une attention particulière a été portée à de potentiels phénomènes immunologiques.

L'étude a été menée comme suit :

| Groupe/ traitement | Dose⁽¹⁾ (µg/kg / adm) | Volume de dose (mL/kg / adm) | Concentratio n de dose (µg/mL) | Nombre d'animaux | | | |
|---|---|---|---|---|---|---|---|
| | | | | Après traitement⁽²⁾ | | Après période d'observation⁽³⁾ | |
| | | | | male | femelle | male | femelle |
| | | | | s | s | s | s |
| 1. Contrôle (véhicule : 0,9 % NaCl) | 0,0 | 4,0 | 0,0 | 10 | 10 | 5 | 5 |
| 2. faible dose | 16,8 | 0,6 | 28,0 | 10 | 10 | / | / |
| 3. dose intermédiair e | 33,6 | 1,2 | 28,0 | 10 | 10 | / | / |
| 4. forte dose | 112,0 | 4,0 | 28,0 | 10 | 10 | 5 | 5 |

| | | | | | | | |
|---|---|---|---|---|---|---|---|
| (1) Exprimé en protéines (2) Rats tués 2 jours après la dernière administration (23^{ième} jour) (3) Rats tués 4 semaines après la dernière administration (49^{ième} jour) | | | | | | | |

Pendant la période de traitement, les rats ont été observés avant et au moins 3 fois après l'administration. Pendant la période d'observation, les rats étaient observés une fois par jour.

Un examen clinique complet était réalisé une fois par semaine. La tolérance locale a été notée avant et après chaque injection, une fois par jour durant la première semaine et ensuite deux fois par semaine jusqu'à la fin de la période d'observation. La température corporelle a été mesurée le lendemain de chaque administration (jours 1, 8, 15 et 22).

Des examens ophtalmologiques ont été réalisés avant le test, ainsi que un jour après le premier et le dernier jour d'administration (jours 1 et 22).

Les poids corporels individuels et la consommation de nourriture ont été mesurés 2 fois par semaine.

Des analyses hématologiques, de coagulation, des paramètres de la chimie clinique sérique et de lymphocites ont été réalisés les 23^{ème} et 49/48^{ème} jours (male/femelle respectivement). Des analyses d'urine ont été effectuées le 23^{ème} jour.

Tous les animaux ont été tués 2 jours après la dernière administration ou après une période d'observation de 4 semaines et autopsiés. Certains organes ont été pesés et utilisés pour les examens histopathologiques.

Les résultats de l'étude ont montré que :
- aucune mortalité n'est observée quelle que soit la dose ;
- l'administration sous-cutanée à des doses exprimées en protéines de 16,8, 33,6 et 112,0 µg/kg/adm a été bien tolérée localement ;
- aucun signe clinique lié au traitement n'est observé ;
- la température corporelle n'est pas affectée ;
- aucun impact ophtalmologique lié au traitement n'a été observé ;
- le poids corporel et la consommation alimentaire ne sont pas affectés ;
- aucune différence significative en ce qui concerne l'hématologie, la coagulation et l'urine n'a été observée ;
- l'activité enzymatique des créatine-kinases a diminué dans les groupes 3 et 4 de femelles (doses intermédiaire et forte ; 33,6 et 112 µg/kg/adm respectivement) à la fin de la période de traitement. Ces variations n'ont pas été considérées comme préjudiciables dans la mesure où elles ne sont pas associées à des caractéristiques histopathologiques ;
- une légère diminution du nombre de cellules NK circulantes chez les femelles traitées et chez les males traités avec des doses intermédiaire et forte (groupes 3 et 4) ;
- à la fin de la période d'observation, la valeur moyenne du nombre de cellules NK circulantes chez les males traités à forte dose (groupe 4) était plus faible ;
- aucune modification du poids des organes n'a été observée ;
- une tâche sombre a été observée au niveau du lieu d'injection, ce qui correspond à des hémorragies sous-cutanées durant l'évaluation histopathologique. Cette observation est considérée comme non liée à la procédure d'administration.

A la fin du traitement, des changements inflammatoires cutanés minimes ou légers au lieu d'injection des groupes 3 et 4, tandis que quasi aucun changement inflammatoire n'a été observé pour les groupes 1 et 2.

A la fin de la période d'observation, les changements inflammatoires observés précédemment pour le groupe 4 ont montré un rétablissement quasi-total.

Les 4 injections sous-cutanées de l'extrait de feuilles de tabac selon l'exemple 1 jusqu'à une dose de 112 µg/kg/adm ont été bien tolérées et seulement de minimes et réversibles changements inflammatoires sont apparus chez les rats traités comparativement aux rats non traités.

En conclusion, dans les conditions expérimentales définies ci-dessus, l'administration sous-cutanée dans des rats Wistar de l'extrait de feuilles de tabac selon l'exemple 1 une fois par semaine pendant 4 semaines, à des doses de 16,8, 33,6 et 112 µg/kg/adm, a été bien tolérée et n'est pas associé à un quelconque effet indésirable.

### Exemple 7 : étude clinique suite à l'administration d'un extrait de feuilles de tabac selon l'exemple 1

Une composition pharmaceutique comprenant de l'extrait de feuille de tabac selon l'exemple 1, contenant 100 µg ou 200 µg de protéines et du mannitol, a été administrée par injection sous cutanée une première fois à J0 et une seconde fois un mis après à J29 (1 mL dans chaque bras à chaque fois) à 24 fumeurs. Les inclusions ont été réalisées suivant le principe d'extension de cohortes, 6 premiers patients recevant 100 µg étant inclus en premier, suivis de 6 premiers patients recevant 200 µg, avant l'inclusion de 6 patients supplémentaires recevant 100 µg. La population de sujets traités était constituée de 24 personnes, 14 hommes et 10 femmes, d'âge compris entre 30 et 65 ans avec un âge médian de 44,5 ans.

Il convient de noter qu'aucune des doses testées n'a généré une toxicité de niveau 3-4 selon les grades de toxicité de la classification Common Terminology Criteria for Adverse Events (CTCAE) v 4.0 du Na tional Cancer Institute.

Les caractéristiques initiales des sujets en matière de consommation de tabac sont mentionnées dans le tableau ci-dessous.

| | | **N** | **%** | **Médiane** | **Etendue** |
|---|---|---|---|---|---|
| **Statut du consommateur** | | | | | |
| | Fumeur | 24 | 100 | | |
| **Consommation** | | | | | |
| | Cigarette manufacturées, Nb/J | 18 | 75 | 20 | (15 ; 30) |
| | | 3 | 12.5 | 20 | (15 ; 30) |
| | Cigarettes roulées, Nb/J | 3 | 12.5 | 20 | (15 ; 20) |
| | Cigarillos, Nb/J | | | | |

| **Niveau de dépendance Fagerstom** | | | | | |
|---|---|---|---|---|---|
| | 5 | 11 | 46 | | |
| | 6 | 5 | 21 | | |
| | 7 | 2 | 8 | | |
| | 8 | 3 | 12.5 | | |
| | 9 | 3 | 12.5 | | |
| **Mesure du CO exhalé (ppm)** | | 24 | | 24.5 | (9 ; 45) |
| **Souhaitez-vous arrêter de fumer ?** | | | | | |
| | Non | 24 | 100 | | |
| | Oui | | | | |

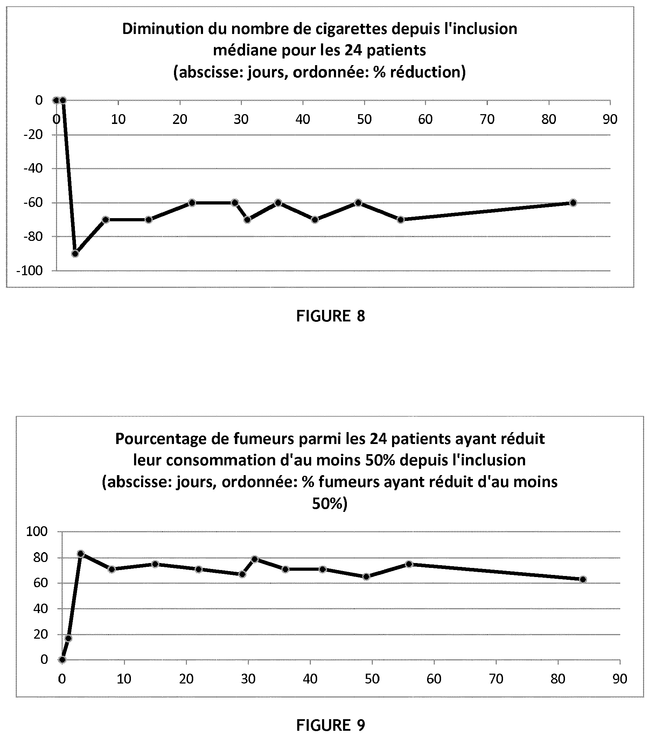

Les figures 8 et 9 montrent l'évolution de la consommation de tabac.

La formule de calcul de la diminution du nombre de cigarettes s'établit comme suit : (Nb de cigarettes visite n - Nb de cigarettes à l'inclusion) / Nb de cigarettes à l'inclusion.

Un point fait à 4 semaines a mis en évidence que 16 fumeurs (soit 67 % des fumeurs) avaient arrêté de fumer ou réduit leur consommation quotidienne de cigarettes d'au moins 50 % et que la consommation de cigarettes quotidiennes des 24 fumeurs avait diminué de 60 %.

Un point fait à 12 semaines a mis en évidence que 15 fumeurs (soit 63 % des fumeurs) avaient arrêté de fumer ou réduit leur consommation quotidienne de cigarettes d'au moins 50 % et que la consommation de cigarettes quotidiennes des 24 fumeurs avait diminué de 60 %.

Il est intéressant de relever que les 12 derniers fumeurs (soit 12 sur 24) ont bénéficié d'un délai plus long (parfois d'un mois ou plus) entre leur première prise de contact avec le centre d'étude clinique et l'injection d'un extrait de tabac selon l'exemple 1 alors que les 12 premiers fumeurs ont bénéficié d'un délai plus court (souvent de quelques jours seulement). Pour ces 12 fumeurs ayant bénéficié d'un délai plus long, le pourcentage de fumeurs ayant réduit d'au moins 50 % ou arrêté leur consommation à 3 et 4 semaines est de 83 % (10 fumeurs sur 12) alors qu'il est de 50 % (6 fumeurs sur 12) pour les 12 fumeurs ayant bénéficié d'un délai plus court. Cette efficacité supérieure s'explique par la meilleure préparation de ces fumeurs qui ont eu plus de temps pour renforcer leur motivation avant de recevoir une injection d'un extrait de tabac selon l'exemple 1. Sur ces 12 derniers fumeurs, 5 étaient abstinents continus depuis une semaine après la fin de traitement jusqu'à la semaine 12 (fin du suivi) et 8 étaient abstinents continus sur les 7 derniers jours à la semaine 12.

Les fumeurs traités décrivent une réduction de leur attirance pour la cigarette et de leur désir de fumer ainsi que l'instauration d'une certaine indifférence à la cigarette. Ils notent une réduction de l'appétence des cigarettes. Certains parlent d'un changement de goût des cigarettes. Leur motivation à arrêter est renforcée.

Avant administration de la composition pharmaceutique des anticorps IgG dirigés contre la composition pharmaceutique ont été détectés chez tous les patients. Avant traitement les concentrations moyennes d'IgG dirigées contre la composition pharmaceutique étaient de 6,07 microg/ml (écart type : 2,98 microg/ml).

Après administration de la composition pharmaceutique, une augmentation significative des concentrations d'IgG anti- composition pharmaceutique a été observée. Ainsi, les concentrations d'IgG mesurées 29 jours après l'administration de la composition pharmaceutique étaient de 7,19 microg/ml (écart type : 4,04 microg/ml) et ces concentrations augmentaient à 7,5 microg/ml (écart type 4,28 microg/ml) lorsqu'elles étaient dosées 8 semaines après la première administration de la composition pharmaceutique. Les variations des concentrations d'IgG étaient significatives (t-test seuil de significativité p<0.05) entre J29 et J0 (p = 0.02) et entre les dosages de la semaine 8 et le J0 (p = 0.006). Une différence significative était également observée entre les concentrations d'IgG anti- composition pharmaceutique à la semaine 8 et à J29 (p = 0.029).

L'analyse statistique croisant les concentrations d'IgG dirigées contre la composition pharmaceutique et le sevrage tabagique a mis en évidence des corrélations (test de Wilcoxon, seuil de significativité p<0.05) entre des niveaux plus élevés d'IgG dirigés contre la composition pharmaceutique et le sevrage tabagique.

Les patients qui ont réduit leur consommation d'au moins 50 % une semaine après la première administration de la composition pharmaceutique ont eu des taux plus élevés d'IgG dirigées contre la composition pharmaceutique à J29 (p=0.034) et à la semaine 8 (p=0.044).

Les patients qui ont été abstinents sur 7 jours consécutif (abstinence prévalente) à J36 ont eu des taux plus élevés d'IgG dirigées contre la composition pharmaceutique à J29 (p<0.001) et à la semaine 8 (p<0.001).

Les patients qui ont été abstinents sur 7 jours consécutif (abstinence prévalente) à la semaine 12 ont eu des taux plus élevés d'IgG dirigées contre la composition pharmaceutique à J29 (p=0.013) et à la semaine 8 (p=0.016).

Les patients qui ont été abstinents continus jusqu'à la semaine 12 ont eu des taux plus élevés d'IgG dirigées contre la composition pharmaceutique à J29 (p=0.007) et à la semaine 8 (p=0.009).

Il n'a pas été détecté d'anticorps de type IgE suite à l'administration de la composition pharmaceutique.

## Revendications

1. Kit comprenant des doses d'extrait de feuilles de tabac ou une composition pharmaceutique comprenant un excipient pharmaceutiquement acceptable et à titre de principe actif un extrait de feuilles de tabac, **caractérisé en ce que** ledit extrait de feuilles de tabac contient au moins 5 % en poids, par rapport au poids total de l'extrait sec, de protéines de masse moléculaire supérieure à 10 kDa et est essentiellement exempt de molécules de masse moléculaire inférieure à 10 kDa, lesdites protéines étant de préférence choisies parmi le groupe constitué des familles de protéines suivantes : peroxidase anionique formant de la lignine, glucan endo-1 ,3-béta-glucosidase, endochitinase, protéine liée à la pathogénèse, osmotine et inhibiteur de protéinase ainsi que leurs mélanges ;
ledit extrait de feuilles de tabac étant **caractérisé en ce que** :
- la teneur en protéines dont la masse moléculaire est supérieure à 500 kDa, de préférence dont la masse moléculaire est supérieure à 400 kDa, de préférence encore dont la masse moléculaire est supérieure à 300 kDa, de manière préférentielle dont la masse moléculaire est supérieure à 200 kDa, de manière plus préférentielle dont la masse moléculaire est supérieure à 150 kDa, de manière plus préférentielle encore dont la masse moléculaire est supérieure à 100 kDa et mieux encore dont la masse moléculaire est supérieure à 50 kDa, est inférieure à 15% en poids par rapport au poids protéique total de l'extrait, de préférence inférieur à 10% en poids par rapport au poids protéique total de l'extrait, de préférence encore inférieure à 7,5% en poids par rapport au poids protéique total de l'extrait, de préférence encore inférieure à 5% en poids par rapport au poids par rapport au poids protéique total de l'extrait, de manière préférentielle inférieure à 2,5 % en poids par rapport au poids protéique total de l'extrait, de manière plus préférentielle encore inférieure à 1% en poids par rapport au poids protéique total de l'extrait, et mieux encore inférieur à 0,5% en poids par rapport au poids protéique total de l'extrait ; et
- la teneur en molécules de masse moléculaire inférieure à 10 kDa est inférieure à 5% en poids par rapport au poids total de l'extrait, de préférence inférieure à 2,5% en poids, et mieux encore inférieure à 1% en poids.

2. Kit selon la revendication 1, **caractérisé en ce que** ledit extrait de feuilles de tabac est susceptible d'être obtenu par un procédé d'extraction par un solvant, par exemple un solvant aqueux, d'un broyât de feuilles de tabac séchées suivie d'une séparation des résidus solides de la solution d'extrait de broyât de feuilles de tabac séchées puis d'une diafiltration à volume constant de la solution d'extrait de broyât de feuilles de tabac séchées sans résidu solide avec un solvant aqueux en une quantité allant de 2 à 12 fois en volume, de préférence 3 à 10 fois en volume, de préférence 4 à 8 fois en volume, de préférence 6 fois en volume, par rapport au volume de l'extrait et avec une membrane ayant un seuil de coupure à 10 kDa.

3. Kit selon la revendication 1 ou 2, **caractérisé en ce que** ledit extrait de feuilles de tabac comprend au moins une protéine appartenant à la famille des glucan endo-1 ,3-béta-glucosidases, et de préférence choisie(s) parmi l'isoforme acide PR-Q' de la béta-1 ,3-endoglucanase (PR36401 selon la base UniProt), l'isoforme vacuolaire basique GLB de la béta-1,3-endoglucanase (P27666 selon la base UniProt), et leurs mélanges.

4. Kit selon l'une quelconque des revendications précédentes, **caractérisé en ce que** ledit extrait de feuilles de tabac comprend au moins une protéine appartenant à la famille des endochitinases, et de préférence choisie(s) parmi l'endochinitase P acide (P17513 selon la base UniProt), l'endochinitase Q acide (P17514 selon la base UniProt), l'endochinitase B (P24091 selon la base UniProt), et leurs mélanges.

5. Kit selon l'une quelconque des revendications précédentes, **caractérisé en ce que** ledit extrait de feuilles de tabac comprend au moins de l'osmotine (P14170 selon la base UniProt).

6. Kit selon l'une quelconque des revendications précédentes, **caractérisé en ce que** ledit extrait de feuilles de tabac comprend au moins une peroxidase anionique formant de la lignine (P11965 selon la base UniProt).

7. Kit selon l'une quelconque des revendications précédentes, **caractérisé en ce que** ledit extrait de feuilles de tabac comprend au moins une protéine liée à la pathogénèse, et de préférence choisie(s) parmi la protéine R liée à la pathogénèse (P13046 selon la base UniProt), la protéine PR-4A liée à la pathogénèse (PR29062 selon la base UniProt), la protéine PR-4B liée à la pathogénèse (PR29063 selon la base UniProt), et leurs mélanges.

8. Kit selon l'une quelconque des revendications précédentes, **caractérisé en ce que** ledit extrait de feuilles de tabac comprend au moins une protéine appartenant à la famille des inhibiteurs de protéinase, et de préférence choisie(s) parmi l'inhibiteur de protéinase I-B (Q03199 selon la base UniProt), l'inhibiteur de protéinase I-A (Q03198 selon la base UniProt), et leurs mélanges.

9. Kit selon au moins l'une quelconque des revendications précédentes, **caractérisé en ce que** la teneur en protéines dans l'extrait sec est d'au moins 10 % en poids, de préférence au moins 15 % en poids, de préférence au moins 20 % en poids par rapport au poids total de l'extrait sec.

10. Kit selon au moins l'une quelconque des revendications précédentes, **caractérisée en ce que** les protéines présentes dans ledit extrait de feuilles de tabac sont présentes dans la composition pharmaceutique en une quantité allant de 1 à 1 000 µg/mL, de préférence de 10 à 500 µg/mL, de préférence de 50 à 300 µg/mL, de préférence de 60 à 200 µg/mL, de préférence entre 80 et 150 µg/mL.

11. Kit selon au moins l'une quelconque des revendications précédentes, **caractérisée en ce que** la composition pharmaceutique contient en outre des protéines extraites du cannabis.

12. Kit selon au moins l'une quelconque des revendications précédentes, **caractérisée en ce que** la composition pharmaceutique c est présentée sous une forme propre à une administration par injection sous cutanée, ou sous une forme propre à une administration à l'aide d'un système thérapeutique transdermique adhésif tel qu'un patch, ou sous une forme propre à une administration par pulvérisation ou par vaporisation.

13. Kit selon au moins l'une quelconque des revendications précédentes, **caractérisée en ce que** le kit comprend une ou des dose(s) d'extrait de feuilles de tabac, de préférence sous forme lyophilisée, ainsi qu'une ou des dose(s) de sérum physiologique ou d'eau PPI.

14. Kit selon au moins l'une quelconque des revendications précédentes, **caractérisée en ce que** le kit comprend une ou des seringue(s) et/ou un ou des patch(s) pour l'administration de la composition pharmaceutique.

15. Kit selon au moins l'une quelconque des revendications précédentes, pour son utilisation dans le traitement de l'addiction au tabac, ou pour son utilisation dans le traitement conjoint de l'addiction au tabac et au cannabis.

16. Kit selon au moins l'une quelconque des revendications précédentes pour son utilisation dans le traitement de l'addiction au tabac, ou dans le traitement conjoint de l'addiction au tabac et au cannabis selon la revendication 15, **caractérisée en ce que** la composition pharmaceutique est présentée sous une forme de dosage de 0,03 mL à 10 mL, de préférence de 0,1 mL à 5 mL, de préférence de 0,5 à 2 mL.
